# EUROPEAN PATENT APPLICATION

(11) **EP 1 741 790 A1**
(43) Date of publication of application: **10.01.2007**
(21) Application number: 05006552.3
(22) Date of filing: 24.03.2005
(51) Int. Cl.: C12Q 1/37

(54) **Methods to identify and characterize prolyl oligopeptidases by using substrates and products thereof**

(71) Applicant: DIGILAB BioVisioN GmbH, 30625 Hannover (DE)
(72) Inventor: Jost, Marco, 53127 Bonn (DE); Lamerz, Jens, 30169 Hannover (DE); Lamping, Norbert, 30175 Hannover (DE); Budde, Petra, 30655 Hannover (DE); Rose, Horst, 31303 Burgdorf (DE); Jürgens, Michael, 30629 Hannover (DE); Schulz-Knappe, Peter, 30966 Hemmingen (DE); Zucht, Hans-Dieter, 30539 Hannover (DE)
(74) Representative: Gramm, Werner

(57) **Abstract**

Proteases such as those belonging to the prolyl oligopeptidase family (POP-family), e.g. DPP4 and DPP8, are involved in many pathological processes and diseases such as diabetes, obesity, hypotension and inflammatory diseases and therefore, these proteases are attractive drug targets. Especially inhibitors of DPP4 and/or DPP8 bear great potential as drug candidates and consequently screening assays and assays to identify and characterize the properties of these potential drugs are of very high interest as valuable tools for drug research. The invention provides such screening assays as well as a panel of peptides or proteins which are substrates or products of a protease of the POP-family and which consequently can be used in various tests to determine the activity of said protease, to screen for inhibitors of said protease and to analyze the specificity of different inhibitors of said protease in clinical studies.

SEQUENCE LISTING

<110>  BioVisioN AG

<120>  Methods to identify and characterize prolyl oligopeptidases by using
substrates and products thereof

<130>  412-11

<160>  9

<170>  PatentIn version 3.1

<210>  1
<211>  19
<212>  PRT
<213>  Rattus norvegicus

<220>
<221>  MISC_FEATURE
<223>  3 out of the 4 proline residues present in the sequence represent
        hydroxyproline

<400>  1

Gly Thr Ala Gly Glu Pro Gly Lys Ala Gly Glu Arg Gly Val Pro Gly
1               5                   10                  15

Pro Pro Gly

<210>  2
<211>  25
<212>  PRT
<213>  Rattus norvegicus

<220>
<221>  MISC_FEATURE

<223>  4 out of the 6 proline residues present in the sequence represent
       hydroxyproline

<400>  2

Leu Pro Gly Pro Pro Gly Glu Arg Gly Gly Pro Gly Ser Arg Gly Phe
1               5                   10                  15

Pro Gly Ala Asp Gly Val Ala Gly Pro
            20                  25

<210>  3

<211>  15

<212>  PRT

<213>  Rattus norvegicus

<220>

<221>  MISC_FEATURE

<223>  2 out of the 4 proline residues present in the sequence represent
       hydroxyproline

<400>  3

Pro Pro Gly Pro Pro Gly Leu Gly Gly Gly Asn Phe Ala Ala Gln
1               5                   10                  15

<210>  4

<211>  17

<212>  PRT

<213>  Rattus norvegicus

<220>

<221>  MISC_FEATURE

<223>  4 out of the 8 proline residues present in the sequence represent
       hydroxyproline

<400>  4

Pro Pro Gly Pro Pro Gly Pro Pro Gly Pro Pro Gly Val Ser Gly Gly
1               5                   10                  15

Gly

<210>  5

<211>  26

<212>  PRT

<213>  Rattus norvegicus



<400>  5

Gly Ala Gly Ser Ser Arg Pro Ser Ser Gly Ser Gly Asn Leu Lys Pro
1               5                   10                  15

Ser Asn Pro Asp Trp Gly Glu Phe Ser Glu
            20                  25

<210>  6

<211>  36

<212>  PRT

<213>  Rattus norvegicus



<220>

<221>  MISC_FEATURE

<223>  either 8 out of 9 proline residues present in the sequence repres
       ent hydroxyproline or 7 out of 9 proline residues present in the
       sequence represent hydroxyproline and the methionine residue repr
       esents oxidized methionine



<400>  6

Val Ala Gly Glu Pro Gly Arg Asp Gly Thr Pro Gly Gly Pro Gly Ile
1               5                   10                  15

Arg Gly Met Pro Gly Ser Pro Gly Gly Pro Gly Asn Asp Gly Lys Pro
            20                  25                  30

Gly Pro Pro Gly
        35

<210>  7

<211>  28

<212>  PRT

<213>  Rattus norvegicus



<220>

<221>  MISC_FEATURE

<223>  4 out of the 5 proline residues present in the sequence represent
       hydroxyproline

<400>  7

Gly Ala Pro Gly Gln Asn Gly Glu Pro Gly Ala Lys Gly Glu Arg Gly
1               5                   10                  15

Ala Pro Gly Glu Lys Gly Glu Gly Gly Pro Pro Gly
            20                  25

<210>  8

<211>  28

<212>  PRT

<213>  Rattus norvegicus

<220>

<221>  MISC_FEATURE

<223>  4 out of the 7 proline residues present in the sequence represent
       hydroxyproline

<400>  8

Pro Pro Gly Ala Asp Gly Gln Pro Gly Ala Lys Gly Glu Pro Gly Asp
1               5                   10                  15

Thr Gly Val Lys Gly Asp Ala Gly Pro Pro Gly Pro
            20                  25

<210>  9

<211>  46

<212>  PRT

<213>  Rattus norvegicus

<220>

<221>  MISC_FEATURE

<223>  5 out of the 9 proline residues present in the sequence represent
       hydroxyproline

<400>  9

Val Gln Gly Gly Lys Gly Glu Gln Gly Pro Ala Gly Pro Pro Gly Phe

1                       5                             10                            15

Gln Gly Leu Pro Gly Pro Ser Gly Thr Ala Gly Glu Val Gly Lys Pro
                20                            25                            30

Gly Glu Arg Gly Leu Pro Gly Glu Phe Gly Leu Pro Gly Pro
            35                            40                            45

## Description

### Field of the invention

The present invention is in the field of pharmaceutical research tools for identification and characterization of compounds and compositions altering the activity of proteases belonging to the prolyl oligopeptidase family (POP-family), in particular DPP4 (dipeptidyl-peptidase IV DPPIV) and DPP8 (dipeptidyl-peptidase VIII). Specifically, the invention provides substrates and products of proteases belonging to the POP-family, in particular DPP4 and DPP8, as well as methods to use these substrates and products for assaying the activity of proteases belonging to the POP-family. Furthermore, the invention provides methods for screening and identifying compounds or compositions which inhibit or activate proteases belonging to the POP-family and methods for determining differences between compounds or compositions with respect to their effects on proteases belonging the POP-family.

### Background of the invention

The prolyl oligopeptidyl peptidase family (POP-family) of serine proteases consists of four subfamilies, namely of the prolyl oligopeptidase subfamily, the dipeptidyl-peptidase 4 subfamily, the acylaminoacyl-peptidase subfamily and the glutamyl endopeptidase subfamily (Nucleic Acids Res, 2004:32 Database issue, D160-D164) (merops peptidase database, release 6.90, Welcome Trust Sanger Institute, Cambridge, UK; http://merops.sanger.ac.uk). Members of the dipeptidyl-peptidase 4 subfamily among others are DPP4, DPP8, DPP9, dipeptidyl-peptidase homologue DPP6, dipeptidyl-peptidase homologue DPP10, dipeptidyl aminopeptidase A, dipeptidyl aminopeptidase B and prolyl tripeptidyl-peptidase. Most members of the POP protease family are active mainly on oligopeptides of less than 30 amino acids in length, and cleave prolyl bonds resulting in degradation of biologically active peptides.

The prolyl oligopeptidase inhibitor bis(4-acetamidophenyl)-1-(S)-prolylpyrrolidine-2(R,S)-phosphonate (=AB192) is mainly specific for DPP4 (J Med Chem, 1999, 42:1041-52), but AB192 may also show cross-inhibition for other closely related proteases such as DPP8. Both, DPP4 and DPP8 belong to the same subfamily of the POP-family, namely to the dipeptidyl-peptidase 4 subfamily. However, in contrast to DPP4, there is not much known about the biological function of DPP8.

DPP4, which is also termed CD26, intestinal adenosine desaminase complexing protein 2, adcp2, adabp, tp103, thymocyte-activating molecule, THAM or GP110 glycoprotein, is a multifunctional cell surface serine protease with broad tissue expression. A soluble form of DPP4 is released into the circulation upon proteolytic cleavage from the cell surface (= shedding) and selectively removes the N-terminal dipeptide from a variety of peptides with proline or alanine at the second sequence position. DPP4 cleaves peptide bonds C-terminal to a proline (Pro) residue. The consensus sequence of DPP4 is H₂N-Xaa-Pro-Xaa-COOH, wherein proline (Pro) can be replaced by hydroxyproline, alanine, serine, glycine, valine, threonine or leucine and wherein Xaa represents any amino acid residue except proline, hydroxyproline or N-methyl glycine. At the amino-terminal position (H₂N-), DPP4 accepts all amino acid residues, provided that they have a protonated amine group. In general, hydrophobic aliphatic residues are favoured at the amino-terminal position. Substitution of the hydroxyl group of serine or threonine with a phosphate group prevents cleavage of the substrate. Catalysis by DPP4 is strongly stereo-specific. The scissile and P₂-P₁ bonds must be in trans configuration. DPP4 is reported to display weak endopeptidase activity and is likely to be able to digest certain N-blocked peptides and denatured collagen at intramolecular sites (DeMester et al.; 2003 Dipeptidyl Peptidase IV Substrates in Dipeptidyl Aminopeptidases in Health and Disease (Hildebrandt M. et al., eds.), Kluwer Academic/Plenum Publishers). Glucose-dependent, insulin secretion-stimulating hormones, known as incretins (GLP-1: Glucagon-Like Peptide-1 and GIP: Glucose-dependent Insulinotropic Polypeptide) secreted in the digestive tract following meals are rapidly hydrolyzed and inactivated by DPP4. When the hydrolysis by DPP4 is suppressed, for example by specific or unspecific DPP4 inhibitors, the action of incretin (GLP-1 and GIP) is enhanced, which in turn increases the glucose-stimulated secretion of insulin from the beta-cells of the pancreas. This has been shown to improve hyperglycemia in the oral glucose tolerance test (Diabetologia, 1999, 42:1324-31). In addition, GLP-1 is known to be involved in the suppression of appetite and food intake. GLP-1 has also been reported to protect the beta-cells of the pancreas by enhancing cell differentiation and proliferation. Thus, DPP4 inhibitors may be useful therapeutic or preventive agents for diseases associated with GLP-1 and/or GIP, such as obesity and diabetes mellitus. Furthermore, there are many reports suggesting an involvement of DPP4 activities in various diseases such as AIDS (Science, 1993, 262:2045-2050), osteoporosis (Clin Chem, 1988, 34:2499-2501), intestinal disorders (Endocrinology, 2000, 141:4013-4020), diabetes mellitus, obesity, hyperlipidemia (Diabetes, 1998, 47:1663-1670; Life Sci, 2000, 66:91-103), angiogenesis (Agents and Actions 1991, 32:125-127), fertility (WO 00/56296), inflammatory diseases, autoimmune diseases, chronic rheumatoid arthritis (J Immunol, 2001, 166:2041-2048), and cancer (Br J Cancer, 1999, 79:1042-8; J Androl, 2000, 21:220-6).

Commonly, DPP4 activity assays use chromogenic DPP4 substrates. However, the sample to be tested is diluted by adding substrates, diluents and other substances required for these assays. This alters the steady state of the proteolytic reaction catalyzed by DPP4 and consequently influences the DPP4 activity value determined. Therefore, in one aspect it would be desirable to provide a reliable method by which the activity of a protease belonging to the POP-family can be determined without adding a substrate for said protease.

Susceptibility to DPP4 cleavage has been shown for a wide range of substrates using in vitro assays and DPP4 inhibitors are in advanced stages of clinical evaluation for the treatment of diabetes. Inhibition of DPP4 may be of great benefit for individuals suffering from various diseases, but unspecific inhibition of other proteases of the POP-family, such as DPP8 or DPP9 results in serious side effects (Current Enzyme Inhibition, 2005, 1:65-73). Not much is known about endogenous DPP4 substrates. However, to evaluate a protease as a potential drug target, it is important to identify its substrates and cleavage products. Detailed knowledge about protease substrates has a big impact on the drug development process since specificity of inhibitors and thus limited side effects of the corresponding drugs can be predicted more accurately.

### Summary of the invention

In a first aspect, the invention relates to a method of determining the activity and/or amount of a protease belonging to the prolyl oligopeptidase family (POP-family) in a sample comprising the steps of allowing a protease belonging to the POP-family present in the sample to contact a substrate for a protease belonging to the POP-family and determining the amount of at least one peptide or protein comprising at least one peptide or protein selected from the group consisting of Peptides/Proteins 1 to 106 of Table 1.

In a preferred embodiment, this method further comprises the step of comparing the amount of the at least one peptide or protein comprising at least one peptide or protein selected from the group consisting of Peptides/Proteins 1 to 106 of Table 1 determined with a reference value wherein the reference value is preferably derived from the determination of the amount of said at least one peptide or protein comprising at least one peptide or protein selected from the group consisting of Peptides/Proteins 1 to 106 of Table 1 in a second sample. In even more preferred embodiments of this method of the invention, said second sample contains a known amount and/or activity of said protease belonging to the POP-family.

In certain embodiments of this method of the invention, the step of allowing a protease belonging to the POP-family present in the sample to contact a substrate for a protease belonging to the POP-family comprises the step of adding a substrate for a protease belonging to the POP-family to said sample. However, alternatively or additionally, the substrate may also be present in the sample in situ. The latter embodiments of the method of the invention do not require the addition of substrate to the sample.

In further embodiments of this method of the invention, the sample is present in and/or derived from an organism.

In certain embodiments, the method of determining the activity and/or amount of a protease belonging to the prolyl oligopeptidase family (POP-family) according to the invention may also comprise the step of allowing said protease belonging to the POP-family present in the sample to contact at least one compound and/or composition and determining whether said at least one compound and/or composition has an inhibitory, activating or no effect on said protease belonging to the POP-family.

In a second aspect, the invention relates to a method of determining whether a compound or composition modifies the properties of a protease belonging to the POP-family comprising the steps of contacting a sample comprising said protease belonging to the POP-family with said compound or composition and a substrate for said protease belonging to the POP-family and determining the amount of at least one peptide or protein comprising at least one peptide or protein selected from the group consisting of Peptides/Protein 1 to 106 of Table 1.

In a third aspect, the invention relates to a method of determining whether a compound or composition is effective against a disease or condition comprising the steps of contacting a sample comprising a protease belonging to the POP-family with said compound or composition and a substrate for a protease belonging to the POP-family and determining the amount of at least one peptide or protein comprising at least one peptide or protein selected from the group consisting of Peptides/Protein 1 to 106 of Table 1 wherein a compound or composition that has an inhibitory effect on the activity of said protease belonging to the POP-family indicates that the compound or composition is effective against said disease or condition.

In a preferred embodiment, the method according to this aspect of the invention further comprises the step of determining the inhibitory effect of at least one further compound or composition known to be effective against said disease or condition and comparing the inhibitory effects of said compound or composition the effectiveness of which against said disease or condition is to be determined with said at least one further compound or composition known to be effective against said disease or condition. Preferably, the disease or condition is selected from the group consisting of diabetes type 2, obesity, hyperlipidemia, osteoporosis, fertility disorders, cancer, angiogenesis, AIDS, inflammatory diseases, autoimmune diseases, and chronic rheumatoid arthritis.

In preferred embodiments of the methods according to all aspects of the invention, the step of determining the amount of at least one peptide or protein comprises the step of determining the amount of at least one substrate for a protease belonging to the POP-family and/or at least one product formed by the activity of a protease belonging to the POP-family.

In even further preferred embodiments of the methods according to all aspects of the invention, the substrate for the protease belonging to the POP-family comprises at least one peptide or protein selected from the group consisting of Peptides/Proteins 1 to 20 of Table 1 and/or the product formed by the activity of a protease belonging to the POP-family comprises at least one peptide or protein selected from the group consisting of Peptides/Proteins 21 to 106 of Table 1.

The step of determining the amount of at least one peptide or protein comprising at least one peptide or protein selected from the group consisting of Peptides/Proteins 1 to 106 of Table 1 in the methods according to the invention preferably comprises determining the absolute or relative concentration of said at least one peptide or protein. In further preferred embodiments of the methods according to all aspects of the invention, the step of determining the amount of at least one peptide or protein comprising at least one peptide or protein selected from the group consisting of Peptides/Proteins 1 to 106 of Table 1 is performed in a time dependent manner, whereby the amount of the at least one peptide or protein is preferably determined at a first time and at least a second time.

In the most preferred embodiments of the methods according to all aspects of the invention, the step of determining the amount of the at least one peptide or protein comprising at least one peptide or protein selected from the group consisting of Peptides/Proteins 1 to 106 of Table 1 comprises the step of determining a peptide or protein pattern resulting from allowing said protease belonging to the POP-family present in the sample to contact a substrate for said protease belonging to the POP-family or contacting said sample comprising a protease belonging to the POP-family with a substrate for said protease belonging to the POP-family.

Most preferably, the protease belonging to the POP-family in the methods according to the invention is dipeptidyl peptidase 4 (DPP4) or dipeptidyl peptidase 8 (DPP8).

In a further aspect, the invention relates to peptides or proteins which are substrates for proteases belonging to the POP-family, preferably DPP4 and/or DPP8, or are formed by the activity of proteases belonging to the POP-family, preferably DPP4 and/or DPP8, and more preferably are a fragment of collagen. The amount of the peptides or proteins described therein is preferably reduced or increased in relative or absolute quantities in the plasma of a rat treated with bis(4-acetamidophenyl)-1-(S)-prolylpyrrolidine-2-(R,S)-phosphate or with another inhibitor such as a control inhibitor as used in this study, in comparison to a rat not treated with bis(4-acetamidophenyl)-1-(S)-prolylpyrrolidine-2-(R,S)-phosphate or treated with a control inhibitor. The peptides or proteins described herein preferably comprise at least one peptide or protein selected from the group consisting of Peptides/Proteins 1 to 106 of Table 1. In even more preferred embodiments, the peptides or proteins comprise a sequence selected from the group consisting of SEQ ID NO: 1 to 9 and/or are generated by proteolysis of a peptide bond between Proline-Xaa in the consensus sequence N-terminus-Xaa-Proline-Xaa-C-terminus of a peptide or protein comprising at least one peptide or protein selected from the group consisting of Peptides/Proteins 1 to 106 of Table 1, wherein proline can be replaced by hydroxyproline, alanine, serine, glycine, valine, threonine or leucine and wherein Xaa represents any amino acid except proline, hydroxyproline or N-methyl glycine.

The invention also relates to a nucleic acid which is selected from the group consisting of: (a) a nucleic acid which comprises a nucleic acid sequence encoding a peptide or protein according to the invention or derivative thereof, (b) a nucleic acid which hybridizes with the nucleic acid of (a) under stringent conditions, (c) a nucleic acid which is degenerate with respect to the nucleic acid of (a) or (b), and (d) a nucleic acid which is complementary to the nucleic acid of (a), (b) or (c).

### Brief description of the drawings

Figure 1:
   50 to 800 pmol of neurotensin were introduced into plasma samples, subsequently separated by reversed phase chromatography (RP-chromatography) and those fractions containing neurotensin were analyzed by mass spectrometry as described in the examples. There is a linear correlation between the MALDI mass spectrometry signal intensity (y-axis) of the neurotensin peptide and the amount of the neurotensin peptide added to the plasma sample (x-axis), indicating that MALDI mass spectrometry is suitable to quantitatively determine peptides in complex samples such as plasma.
Figure 2:
   Wister rats were either injected with various amounts of the irreversible DPP4 inhibitor AB192 (8 animals per dosage), control inhibitor (Contr. In.) (8 animals per dosage) or vehicle (4 animals). Blood samples were taken immediately prior and 1 h after injection. Plasma was prepared and used for a DPP4 activity assay. The figure shows the DPP4 activity measured in U/L (Units/litre; y-axis) versus the concentration of inhibitor injected (mg/kg; x-axis) for AB192 (panel A) and Contr.In. (panel B). This indicates that both inhibitors inhibit DPP4 activity in vivo in a dose-dependent manner.
Figure 3:

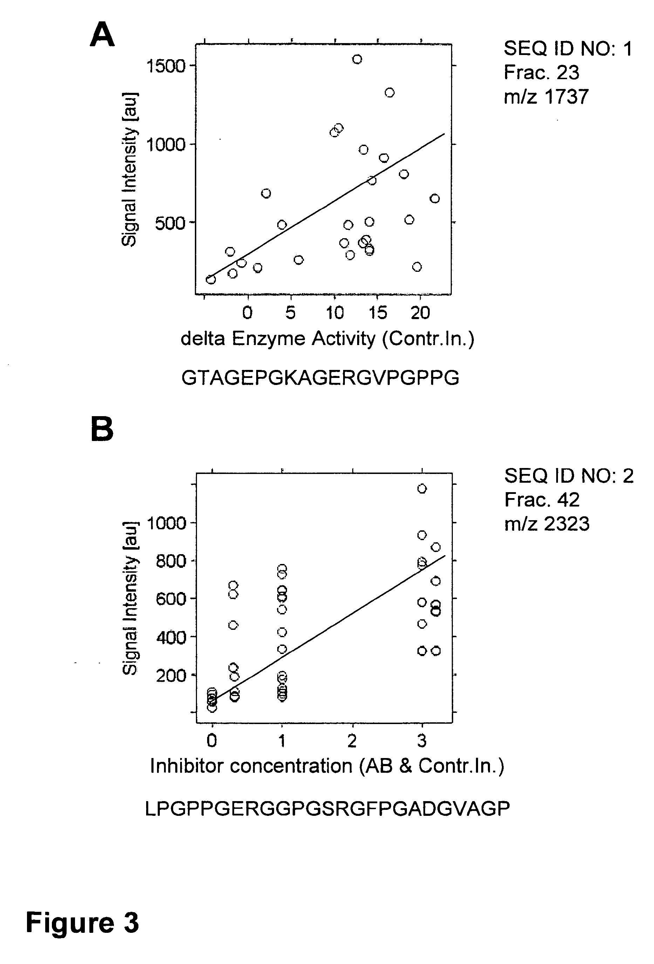

   Figure 3 shows the mass spectrometric signal intensity relative to the DPP4 activity determined in plasma samples of individual rats. Rats were treated with the control inhibitor (Contr. In.). Panel A shows the results of the Peptide / Protein no. 1 identified in fraction 23 (Frac. 23) and having a mass of 1737 (m/z 1737) (Table 1, SEQ ID NO: 1). The correlation shows that the increase in the mass spectrometric signal of Peptide / Protein no. 1 correlates positively and linear with the DPP4 activity measured in the same samples. Panel B shows the results of the Peptide / Protein no. 2 identified in fraction 42 and having a mass of 2323 (Table 1, SEQ ID NO: 2). The correlation shows that the increase in the mass spectrometric signal of Peptide / Protein no. 2 correlates positively and linear with the amount of the DPP4 inhibitor AB192 or the control inhibitor given to the experimental rat. The amino acid sequence of the corresponding peptide / protein is shown below each graph.
Figure 4:

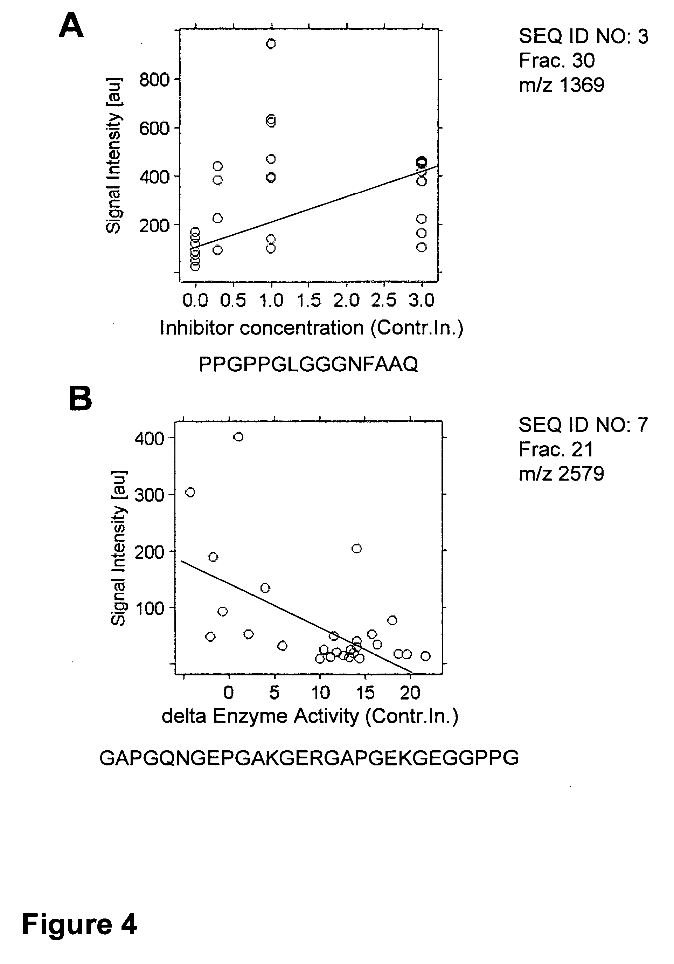

   Figure 4 shows data similar to those presented in Figure 3 for different peptides/proteins. Panel A shows the results of the Peptide / Protein no. 3 identified in fraction 30 and having a mass of 1396 (Table 1, SEQ ID NO: 3). The correlation shows that the increase in the mass spectrometric signal of Peptide / Protein no. 3 correlates positively and linear with the amount of control inhibitor (Contr. In.) given to the experimental rat. Panel B shows the results of the Peptide / Protein no. 45 identified in fraction 21 and having a mass of 2579 (Table 1, SEQ ID NO: 7). The correlation shows that the increase in the mass spectrometric signal of Peptide / Protein no. 45 correlates negatively with the DPP4 activity measured in the same samples of experimental rats treated with the control inhibitor. The amino acid sequence of the corresponding peptide / protein is shown below each graph.
Figure 5:

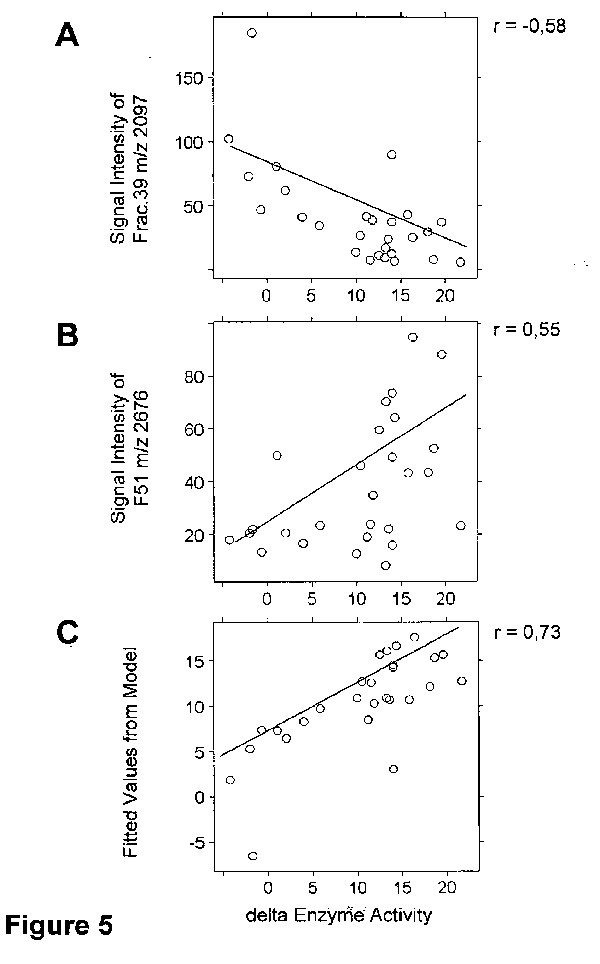

   Figure 5 shows panels A to C each representing combinations of two peptides or proteins from Table 1 (Peptide / Protein no. 56 and 16 of Table 1 form panel no. 62 of Table 2). A combination of these peptides or proteins to form a peptide / protein panel improves the correlation value as compared to the correlation values of the individual peptides / proteins. When used individually Peptide / Protein no. 56 results in a correlation coefficient r of -0.58 (panel A) and Peptide / Protein no. 16 results in a correlation coefficient r of 0.55 (panel B). When combining both peptides / proteins to form a panel, the correlation coefficient r is improved to 0.73 (panel C).

### Detailed description of the invention

### Determining the activity and/or amount of a protease

According to the invention, the activity and/or amount of a protease belonging to the POP-family can be measured by determining the amount of substrate for and/or product formed by the activity of said protease. The more protease is present in a sample or the higher the activity of the protease, the more substrate will be transformed into product by said protease. Accordingly, the amount of substrate determined in a sample will decrease with increasing amount and/or activity of protease in the sample and the amount of product will increase in the sample. Thus, a small amount of substrate and/or large amount of product determined in a sample is indicative for a high amount and/or activity of protease in the sample. A large amount of substrate and/or small amount of product determined in a sample is indicative for a small amount and/or low activity of protease in the sample.

The expression "determining the activity and/or amount of a protease" according to the invention also includes situations wherein no product formed by the activity of said protease is detected or the amount of product formed by the activity of said protease is below the detection limit of a particular test system used which situations are indicative for the fact that no or substantially no protease is present in the sample, protease present in the sample is substantially or completely inactive or the activity of the protease is substantially or completely inhibited.

Furthermore, the expression "determining the amount of at least one peptide or protein comprising at least one peptide or protein selected from the group consisting of Peptides/Proteins 1 to 106 of Table 1" also includes situations wherein said peptide or protein comprising at least one peptide or protein selected from the group consisting of Peptides/Proteins 1 to 106 of Table 1 is not detectable either because it is not present in the sample or because its concentration is below the detection limit of the test system used. In those cases where the peptide or protein determined is a substrate for a protease this situation is indicative for a high amount or activity of protease in the sample whereas in cases where the peptide or protein determined is a product formed by the activity of a protease this situation is indicative for a small amount or low activity of protease in the sample.

The term "peptide or protein comprising a particular peptide or protein" indicates that the peptide or protein comprises the sequence of the latter peptide or protein. Preferably, the peptide or protein consists of the sequence of the latter peptide or protein.

On the basis of the amount of at least one peptide or protein comprising at least one peptide or protein selected from the group consisting of Peptides/Proteins 1 to 106 of Table 1 determined in a sample it is possible in the methods of the invention to draw conclusions about the activity and/or amount of protease in the sample. In this respect, the amount or activity of the protease may be calculated as a relative or an absolute concentration or activity, depending on the experimental setup.

An experimental setup to determine the relative concentration or activity of a protease may be conducted, for example, by measuring the amount or concentration of a substrate of said protease or a product formed by the activity of said protease in an unknown sample. This amount or concentration of a substrate of said protease or a product formed by the activity of said protease is compared with a reference value obtained from a sample having known characteristics such as a known concentration, amount and/or activity of said protease (for example a known molar concentration and/or known quantity of units of activity per ml or mg of sample). Thus, it is possible to e.g. determine the relative concentration or activity of said protease in the unknown sample. The amount of substrate or product can be reflected by a signal intensity in a test system, for example a mass spectrometric signal intensity, an absorption value, fluorescence value, radioactive counts, luminescence value, etc. of an ELISA (enzyme-linked immuno-sorbent assay), a RIA (radio immuno assay), a protein- or DNA-chip assay, a FACS (fluorescence activated cell sorting) analysis, an immune precipitation, a quantitative PCR (polymerase chain reaction) or RT-PCR (reverse transcriptase-PCR) reaction, or densitometric signal intensity of an exposed film (for example from a Western blot, a Northern blot, or an in situ hybridization), etc.

An experimental setup to determine the absolute concentration or activity of a protease may be conducted, for example, by quantitatively determining the concentration of a substrate of said protease or a product formed by the activity of said protease, for example by means of an ELISA or by quantitative mass spectrometric measurement of the mass signal of a peptide which is a substrate or product of said protease. Furthermore, by empirically determining with various known concentrations or various known quantities of activities of said protease the reduction in substrate concentration or increase in product concentration over time, it is possible to establish a standard curve for determining the absolute concentration, quantity and/or activity of said protease in an unknown sample.

If the products (peptides and/or proteins) formed by the activity of a protease belonging to the POP-family are measured, the determination may represent not only the actual activity of said protease present in the sample at the time point of taking the sample, but also may indicate the relative activity of said protease over a certain period of time in the past. In case, the activity of said protease changes rapidly even within short periods of time, the measurement of the activity of said protease at one distinct time point by adding substrates, e.g. chromogenic substrates, to the sample may result in not obtaining relevant activity values for said protease. In this case, measurements of peptides and/or proteins representing products of said protease without adding substrates more accurately indicate the overall activity of said protease present in the sample. In an alternative embodiment, it is also possible to determine the activity of said protease in a time dependent manner instead of determining the activity at one distinct time point.

In addition the same strategy can be used for determining whether a compound or composition modifies the properties of a protease belonging to the POP-family (e.g. whether said compound or composition has an inhibitory or activating effect on the activity of said protease) or for testing whether a compound or composition is effective against a disease or condition, e.g. during treatment of a single patient having said disease or condition or during clinical trials. By measuring the amount or concentration of substrates or products, preferably products, of said protease the overall effectiveness of the compound or composition over time in a patient can be determined. This could be used to monitor the therapy result, to adjust the dose of the compound or composition or to determine the in vivo effectiveness or specificity of said compound or composition on said protease. It is also possible to compare different compounds or compositions with each other using this strategy and/or to screen for compounds (using e.g. in vivo or in vitro systems) that are effective or even more effective against said disease or condition.

### Allowing a protease to contact a substrate or compound and/or composition

The expressions "allowing a protease to contact a substrate" or "allowing a protease to contact a compound and/or composition" refer to the situation that a protease and a substrate for said protease, or protease and said compound and/or composition are present in the same sample and come into contact. For example, the substrate can be present in the sample in situ or it can be added to the sample. The term "in situ" with respect to the substrate according to the invention means that the substrate is not added to the sample. However, the present invention also includes situations wherein a substrate is present in a sample in situ and in addition, the same or a different substrate is added to the sample.

### Substrates, products and labels for substrates and products

According to the invention, a substrate for a protease belonging to the POP-family comprises at least one bond susceptible to cleavage by said protease. Preferably, a substrate for a protease belonging to the POP-family is a peptide or protein. However, such substrate may also comprise non-peptide or non-protein portions. Accordingly, the product formed by the activity of said protease is a peptide or protein which may also include non-peptide or non-protein portions.

Preferably, the substrates and/or products used according to the invention comprise at least one peptide or protein selected from the group consisting of Peptides/Proteins 1 to 106 of Table 1. Said peptides or proteins can be present in the sample in situ, e.g. in vitro cultured tissues or cells, or tissue or body fluid within an organism or individual, or can be added intentionally to the sample.

Peptides or proteins described herein may be of any length. A peptide or protein described herein preferably comprises a sequence of at least 4 or 6, in particular at least 8, at least 10, at least 12, at least 15, at least 20, at least 30 or at least 50 consecutive amino acids.

Preferably, the peptides or proteins described herein have been isolated. The term "isolated peptide or protein" means that the peptide or protein has been separated from its natural environment. An isolated peptide or protein may be in an essentially purified state. The term "essentially purified" means that the peptide is essentially free of other substances with which it is associated in nature or in vivo.

Furthermore, the peptides or proteins described herein may be variants. A "variant" as used herein, comprises a peptide or protein that differs from a peptide or protein from which it is derived, in particular a naturally occurring peptide or protein, in one or more substitutions, deletions, additions and/or insertions of amino acids. Such variants may be those that exist naturally, or those in which one or more substitutions, deletions, additions and/or insertions have been introduced.

Peptide or protein variants according to the invention include those exhibiting at least about 70%, 75%, 80%, 85%, 90%, 91%, 92%, 93%, 94%, 95%, 96%, 97%, 98%, or 99% or more identity to the peptide or protein sequences from which they are derived, in particular the specific peptides and proteins described herein.

The peptides or proteins described herein may also comprise any natural and non-natural modifications, in particular chemical or physical modifications. The term "natural modification" relates to any modification found in peptides or proteins in nature such as posttranslational modifications, modifications due to exposure to light, oxygen, exposure to acid or alkali solutions, etc.

Preferably the peptides or proteins described herein are modified to facilitate their detection, alter their stability during storage or usage (for example by using peptidomimetic structures), alter their toxicity or bioavailability or biological half-life, etc. (for example by pegylation). Preferred modifications are those which facilitate the detection of peptides or proteins such as fluorescent labels, chromophore labels, luminescent labels, radioisotopic labels, isotopic labels, isobaric labels, enzyme labels, particle labels, small organic molecules such as biotin, ligands of receptors or binding molecules such as cell adhesion proteins or lectins, label-sequences comprising nucleic acids and/or amino acids which can be detected by use of binding agents, etc. Label sequences can be bound by binding agents such as antibodies, receptors, PCR-primers, Northern blot probes, etc. Label sequences can be amino acid sequences or nucleic acid sequences or combinations thereof or molecules mimicking the structure of amino acid or nucleic acid sequences (mimetics).

The term "Peptides/Proteins 1 to 106 of Table 1" relates to peptides or proteins which are substrates of a protease belonging to the POP-family or products formed by a protease belonging to the POP-family. Preferably, each of said peptides or proteins has the specific characteristics (e.g. fraction no., m/z value, signal intensity, negative or positive correlation to inhibitor concentration and/or DPP4 activity, sequence) indicated in Table 1 and is identified by subtractive peptide display maps and correlation analysis as described below.

### Labels for peptides and proteins

The invention envisions the use of peptides and proteins, which comprise at least one label. The term "label", as used herein, refers to any moiety that functions to: (i) provide a detectable signal; (ii) interact with a second label to modify the detectable signal provided by the first or second label, e.g. FRET (Fluorescence Resonance Energy Transfer); (iii) affect mobility, e.g. electrophoretic mobility, by charge, hydrophobicity, shape, or other physical parameters, or (iv) provide a capture moiety, e.g., affinity, antibody/antigen, or ionic complexation.

Suitable as label are all kinds of structures, such as isotopic labels, isobar labels, fluorescent labels, radioactive labels, luminescent labels, enzyme labels, toxin labels, dye labels, metal particles as labels, magnetic particles as labels, polymer particles as labels, biotin or other organic molecules as labels, etc.

For example, isotopes with molecular weights different to their natural molecular weight such as oxygen-18, nitrogen-15, deuterium, tritium, carbon-14, sulphur-35, phosphorus-32 or phosphorus-33 etc. can be used to prepare isotope-labelled peptides or proteins. The isotopes can be incorporated directly into the peptides or proteins by using isotope-labelled amino acids for synthesis of the peptides or proteins. Furthermore, these isotopes can be incorporated into peptides or proteins by growing cells in the presence of isotope-labelled metabolites and isolating the labelled peptides or proteins from these cells or cell culture supernatants. The isotopes can also be incorporated into peptides or proteins by other methods such as in vitro translation using isotope-labelled metabolites. Isotope-labelled metabolites can be amino acids, carbohydrates, fatty acids, inorganic salts such as phosphate, sulphate, etc. or other isotope-labelled substances. These isotope-labelled peptides or proteins are especially suitable for measuring methods using mass spectrometric methods.

Another kind of label especially suitable for mass spectrometric detection methods are isobaric labels. Different kinds of such labels have the same molecular weight, as long as these labels are intact and have not been fragmented in a mass spectrometer. This has the advantage, that mixtures of molecules labelled with different isobar labels can more conveniently be analyzed using mass spectrometric methods, as only a limited number of mass differences is present prior to analysis of the sample. Fragmentation of the isobar labels is done to determine the identity of the label, thereby identifying the identity of the molecule labelled with the isobar label. Preferably these labels are constructed in a way that upon fragmentation of the label in a mass spectrometer the fragments generated have masses, which masses usually are not obtained by fragmentation of peptides or proteins. Examples of such masses are masses between 114 and 120 Da. Generally isobaric labels should be selected, which generate masses, which are not generated in the same kind of samples, analyzed by the same mass spectrometric method in the absence of said isobaric labels.

Alternatively, to directly using labelled amino acids in peptide synthesis, peptides and proteins generally can be labelled using amine-reactive reagents such as isothiocyanates, succinimidyl esters and carboxylic acids, sulfonyl chlorides, aldehydes, arylating reagents, etc. or thiol-reactive reagents such as iodoacetamides, maleimides, alkylhalides and arylating agents. Furthermore alcoholes such as those present in serine, threonine or in carbohydrate moieties can be labelled by oxidizing with periodate to yield aldehydes that can be subsequently modified with a variety of amine or hydrazine derivatives. Alcoholes from tyrosine sometimes can be reacted with sulfonyl chlorides or iodoacetamides. Alcoholes in carbohydrate moieties furthermore can be labelled by reaction with dichlorotriazines. N-methylisatoic anhydride will convert ribonucleotides and other carbohydrate moieties to fluorescent esters with excitation/emission maxima of about 350/466 nm. Examples of fluorescent labels are fluorophores such as Alexa Fluor® 350, 405, 430, 488, 500, 514, 532, 546, 555, 568, 594, 610, 633, 635, 647, 660, 680, 700 and Alexa Fluor® 750, Aminomethylcoumarin (AMCA), Methoxy coumarin acetic acid, Bimane, BODIPY 493/503, 530/550, 558/568, 564/570, 576/589, 581/591, 630/650 and BODIPY 650/665, BODIPY FL, BODIPY TR; BODIPY TMR, Cascade Blue dye, Cascade Yellow dye, cyanine dyes such as Cy3, Cy5, Cy5.5, Cy7, Dansyl, Dapoxyl dye, Dialkylaminocoumarin, Eosin, Erythrosin, Fluorescein (FITC), Fluorescein-EX, 2',4',5',T-Tetrabromosulfonefluorescein, Naphthofluorescein, 2',7'-Dichloro-fluorescein, 4',5'-Dichloro-2',7'-dimethoxy-fluorescein, 6-Carboxy-2',4,4',5',7,7'-hexachlorofluorescein, succinimidyl ester (6-HEX, SE), 5-carboxyfluoresceine (5FAM), 5,6-carboxyfluoresceine (5(6)FAM), hydroxycoumarin, Malachite green, Marina Blue dye, methoxycoumarin, 7-nitro-4-benzofurazanyl (NBD), Oregon Green® 488, Oregon Green® 514, Pacific Blue, Pacific Blue dye, PyMPO, Pyrene, QSY 7 , QSY 9, QSY 21, QSY 35, Rhodamine 6G, Rhodamine Green, Rhodamine Green dye, Rhodamine Red dye, Rhodamine Red, Rhodamine Red-X (RRX), X-rhodamine, Tetramethyl-rhodamine (TMR, TRITC), Lissamine rhodamine B, Texas Red, Texas Red dye, Texas Red-X. Most of these fluorophores are available from Molecular Probes, Eugene, OR, USA, and many are also available as kits intended for protein or peptide labelling. Furthermore fluorescent proteins or fragments or derivatives thereof such as green fluorescent protein, red fluorescent protein, blue fluorescent protein, renilla fluorescent protein, etc. can be used as labels. Examples of radioactive isotopes suitable as labels are phosphorous-32 or -33, sulphur-35, iodine-125, tritium, carbon-14, calcium-45, chromium-51 and all other known radioisotopes which can be coupled to nucleic acids and/or peptides or proteins. It is also possible to use matched pairs of fluorescent labels, which are suitable for fluorescence resonance energy transfer (FRET).

Examples of enzyme labels are horse-reddish-peroxidase, alkaline phosphatase, luciferase, galactosidase, etc. Suitable toxin labels are all kinds of toxins such as microbial toxins, toxic peptides or toxic small organic molecules, synthetic toxins or toxins used for medical purposes. An example of suitable dye labels is digoxigenin. Examples of particles as labels are particles of various particle diameters, preferably between 0.1 to 100 µm. Metal particles can be made from various substances, preferably heavy elements such as gold, silver, platinum or other suitable metals or alloys or mixtures thereof. Examples of magnetic particles are preferably made from all kinds of magnetic or magnetizable materials including iron, preferably iron oxides such as Fe₃O, Fe₂O₃, Fe₃O₄, etc. Examples of polymer particles are particles made from a polymer such as polyacryl amide, agarose, polypropylene, polystyrene, polytetrafluoroethylene, fluorescent polystyrene microspheres, etc. Various other labels, such as biotin, chitin, maltose or glutathione etc. as known in the art, can also be used.

Furthermore labels comprising certain additional nucleic acid or amino acid sequences subsequently termed label-sequences can be used. Label-sequences comprising nucleic acid sequences for example can be used for detection of the labelled substance for example by hybridization with a probe specific for the label-sequence or by detection of the label-sequence by polymerase chain reaction, preferably by quantitative polymerase chain reaction, preferably by real-time polymerase chain reaction, etc. Examples of label-sequences comprising amino acid sequences are his-tags, flag-tags, myc-tags or whole proteins or fragments thereof such as antibodies, glutathione S-transferase, streptavidin, chitin-binding protein, maltose binding protein, various lectins, etc. These labels can be detected by use of antibodies or by use of ligands binding to these labels such as protein A, protein G, protein Y, protein A/G, glutathione, biotin, chitin, maltose or other sugars etc. Label-sequences in general can also be used to capture the labelled peptide or protein by a binding agent specific for said label-sequence. Subsequently or during the capture process the labelled peptide or protein can be quantified. Labels can be at the N- or C-terminus or can be present internal in the amino acid sequence of a peptide and/or protein.

### Reference values

The term "reference value" according to the invention refers to a value obtained from a sample having a known amount, activity and/or concentration of protease and/or known amount and/or concentration of a substrate of a protease or product formed by the activity of a protease. Such reference value can be used for comparative purposes with a value obtained from a sample having an unknown amount, activity and/or concentration of protease or unknown amount and/or concentration of a substrate of a protease or product formed by the activity of a protease. In preferred embodiments, the reference value and sample from which it is derived have known characteristics, e.g. it is known that the sample is derived from a healthy or diseased individual. This allows, for example, the conclusion whether the individual from which the unknown sample, i.e. the sample to be tested, is derived has a particular disease/condition or not.

The reference value can be determined empirically by measuring a sufficiently large number of samples. Preferably the reference value is determined by measuring at least 2, preferably at least 3, preferably at least 5, preferably at least 8, preferably at least 12, preferably at least 20, preferably at least 30, preferably at least 50, or preferably at least 100 samples.

### Sample

The term "sample" according to the invention comprises any material which may be used for testing in the methods according to the invention. The sample can be of any source such as humans, animals, plants, microorganisms, in vitro cultured cells or tissues. Preferably the individuals from which the samples are obtained are small laboratory animals such as rats, mice, hamsters, guinea pigs, gerbils, rabbits, zebra fish, drosophila flies or larger mammals, preferably pigs, mini pigs, dogs, cats, apes or humans. It is possible to use tissues such as fat, muscle, blood, bone, intestine, pancreas, liver or tumour tissue or to use in vitro cultured primary cells or cell lines such as fat, muscle, blood, bone, intestine, pancreas, liver or tumour cells or cell culture supernatants from those primary cells or cell lines as samples. The sample can be a present in nature or it can be obtained by use of various techniques such as chemical synthesis, enzymatical synthesis (for example in vitro translation), purification from material found in nature or obtained by use of any of the above noted techniques, etc. Any kind of the above noted samples or combinations thereof can be used according to the invention.

The sample according to the invention, can be present in an organism. This means, that the sample, for example body fluid such as blood, plasma or urine, fat tissue, tumour tissue, etc. is present in the natural environment of the living or dead organism.

### Comparisons between samples

If the methods of the invention are to be applied to more than one sample, in particular to create a mean, the samples can differ from one another, in particular regarding the time point they have been taken from the source which may be the same or different. The difference in time can be more than 1 second, more than 20 seconds, more than 1 minute, more than 5 minutes, more than 10 minutes, more than 30 min., more than 1 hour, more than 2 hours, more than 6 hours, more than 12 hours, more than 1 day, more than 3 days, more than a week, more than a month, or more than a year. Such samples are suitable to perform the methods of the invention in a time-dependent manner.

If the methods of the invention are to be applied to more than one sample, the samples can also differ regarding the source of the samples. For example the samples may be derived from different tissue culture plates, different individuals, different kinds of tissues or cells, different species or organisms of different genetic background (e.g. genetic knock-out versus wild-type, or different ethnic backgrounds), etc. The samples may differ regarding the sex, age, body mass of the organism, from which the samples originate, or regarding the exposure of the organism, from which the samples originate, to chemicals, alcohol, cigarette smoke, etc. The samples may differ regarding the physical activity, regarding the exposure to environmental effects such as access to food, sugar-rich diets, fat-rich diets, protein-rich diets, water, etc. or air pollution, light, humidity, temperature, noise, radiation, psychological stress, etc. of the organism from which the sample originates. The samples my originate from individuals having or not having a disease or other condition, from organisms having different diseases or other conditions or from organisms having different stages of diseases or other conditions. Different stages of a disease or other condition include the time points before, during or after the occurrence or first diagnosis of said disease or other condition. Other conditions among others include pregnancy and fertility. The samples may differ regarding the kind of sample-types such as serum, plasma, whole blood, urine, tissue extracts, etc., obtained from individuals treated or not treated with compounds or compositions. If individuals, cell cultures or samples are treated with compounds or compositions they may be treated for different time intervals, or with different concentrations, or with different compounds or compositions, or with mixtures of two or more compounds or compositions at the same time or at different times, or under different experimental conditions such as temperature, light, humidity, access to nutrients, etc. Samples may also differ by various combinations of the above noted attributes.

Preferably, the results obtained from different samples are compared to each other.

### Peptide and/or protein pattern

The methods of the invention can be performed by generating protein and/or peptide patterns which preferably reflect substrates of a protease and/or products formed by the activity of a protease and preferably comparing protein and/or peptide patterns generated. Such comparison of protein and/or peptide patterns may reflect different amounts of substrates of a protease and/or products formed by the activity of a protease in different protein and/or peptide patterns and thus, be indicative for different amounts and/or activities of a protease in the samples on the basis of which the patterns were established and/or source from which the samples are derived, which in turn could be indicative for the presence or absence of a disease or the presence or absence of a distinct compound or composition which modifies the activity of the protease.

Such protein and/or peptide pattern preferably comprises at least two, preferably three, preferably 4, preferably 5, preferably 6, preferably 7, preferably 8, preferably 9, preferably up to 10, preferably up to 15, preferably up to 20, preferably up to 30, preferably more than 30 different peptides and/or proteins. Preferably such protein and/or peptide pattern comprises at least 1 peptide or protein, preferably 2 , preferably 3 , preferably 4 , preferably 5 or more of the peptides or proteins comprising at least one peptide or protein selected from the group consisting of Peptides/Proteins 1 to 106 of Table 1. For evaluation of such patterns preferably at least one of the following mathematical methods to calculate correlations is used: "Pearson Product-Moment Correlation", "Spearman's Rank-Order Correlation", "Kendall's Tau", "Kendall's Coefficient of Concordance", "Goodman and Kruskal's Gamma", "Manhattan distance", "Euclidean distance" and "Minimal Spanning Tree Diameter". Most preferably "Spearman's Rank-Order Correlation" is used.

Correlations can be calculated for two or more peptides or proteins, between one or more peptides or proteins and additional meta data available, such as POP-family protease activity, DPP4 protease family activity, DPP4 and/or DPP8 activity, concentrations of compounds or compositions, preferably protease inhibitors, preferably POP-family protease inhibitors, most preferably DPP4 or DPP8 inhibitors. The inhibitors can be reversible or irreversible, specific or non-specific inhibitors, specific for POP-family proteases or specific for individual or subgroups of the POP-family of proteases, preferably for the dipeptidyl-peptidase 4 subfamily of the POP-family or specific for DPP4 and/or DPP8. At least one panel of two or more than two peptides or proteins obtained by combining any two peptides or proteins wherein at least one peptide or protein of Table 1 is used, more preferably a panel of two peptides or proteins as shown in Table 2 (panel No 1 to 78; left row of Table 2) is used. With different peptides or proteins is meant all kind of different peptides and proteins present in nature such as different fragments of the same or of different precursor proteins or peptides, the same peptide or protein with different posttranslational, or chemical, or other modifications, etc. The pattern can comprise peptides and proteins with different "behaviour", that means one peptide or protein belonging to the pattern can be increased and another protein or peptide belonging to the pattern can be decreased. So a pattern can be a mixture of peptides and proteins of which some are increased, whereas others are decreased and the complete pattern of increased and decreased peptides and proteins represents said pattern. Therefore the correlation of individual peptides with meta data such as DPP4 activity or concentration of inhibitors can be positive or negative and panels of peptides or proteins can comprise only positive correlating, only negative correlating or positive and negative correlating peptides or proteins (see the right 3 rows of Table 2).

### Inhibitors of proteases of the POP-family

In the methods described herein any compounds or compositions lowering the activity of proteases belonging to the POP-family can be used as inhibitors of proteases of the POP-family.

DPP4 inhibitors can be found, for example, in the merops-database (Nucleic Acids Res, 2004:32 Database issue, D160-D164) http://merops.sanger.ac.uk/): sulphostin, PT-100, cyano-pyrazoline derivatives, substituted piperazines, homophenylalanine 3, ketopyrrolidines, ketoazetidines, 3- or 4-substituted-2-cyanopyrrolidine, diprotin A (= Ile-Pro-Ile), vildagliptin, talabostat, 4-amino cyclohexylglycine analogues, aminomethylpyrimidines, K579, beta-homophenylalanine based dipeptidyl peptidase IV inhibitors, fluoropyrrolidine dipeptidyl peptidase-IV inhibitors, dynorphin-A(1-17), Tat protein, N-terminal nona-peptide of HIV-Tat = Tat(1-9), Tat(1-9) with tryptophan in position 2, N-terminal nona-peptide of thromboxane A2 receptor peptide = TXA2-R(1-9), P32/98, valine-pyrrolidide, TMC-2, 1-[[[2-[(5-cyanopyridin-2-yl)amino]ethyl]amino]acetyl]-2-cyano-(S)-pyrrolidine (= NVP DPP728), FE 999011, 1-[2-[(5-Cyanopyridin-2-yl)amino]ethylamino]acetyl-2-(S)-pyrrolidinecarbonitrile, Ile-thiazolidide, Xaa-psi[CS-N]-pyrrolidide, Xaa-psi[CS-N]-thiazolidide, acyl-2-cyanopyrrolidides, diaryl dipeptide phosphonates, aminoacylpyrrolidine-2-nitriles, N-peptidyl-O-aroyl hydroxylamines, phosphonates, homoprolines, Pro-boroPro (boroPro = amino boronic acid analogue of proline), diprotin A (Ile-Pro-Ile) and diprotin B (Val-Pro-Leu).

### Compound and/or composition

The term "compound and/or composition" according to the invention relates to all kinds of substances including small organic or inorganic molecules, chemically, enzymatically or recombinantly synthesized molecules, such as nucleic acids, peptides, proteins, carbohydrates or lipids, synthetic molecules or molecules found in nature or molecules isolated from materials or organisms found in nature, as well as molecules found in nature with altered structures or with sequences comprising deletions, insertions and mutations of the sequence and combinations or mixtures thereof. Compositions according to the invention comprise at least one compound and additional substances such as saline, diluents, solvents, substances ensuring an appropriate pH, osmolarity or viscosity, additives for preservation, staining, flavouring or scenting, filling substances, anti-microbial, anti-fungal or other preserving agents, substances protecting the compound or composition form light, heat, freezing, or other environmental factors, substances preventing aggregation or protecting from sticking of the compounds or compositions to the container, formulations necessary for specific routes of applications such as gastric acid resistant capsules, or plasters for transdermale application of compounds or compositions etc.

### Effectiveness against a disease or condition

The expression "effective against a disease or condition" means that a compound or composition is appropriate to reduce the risk of an individual treated with said compound or composition to acquire said disease or condition, or heal said disease or condition or stop or slow down the progression of said disease or condition in an individual afflicted therewith.

The expression "modifying the properties of a protease" relates to all changes regarding the properties of a protease such as increasing or decreasing the efficiency of the proteolytic activity of the protease, increasing or decreasing the specificity of the protease for one or more than one substrate of the protease (this may also lead to new substrates or may result in certain substances to be no longer substrates for said protease), increasing or decreasing the biological half-life of the protease, changing the spacial distribution of the protease within an organism, an individual or within a cell, etc.

### Peptides and proteins

The term "peptide" refers to substances consisting of two or more, preferably 3 or more, preferably 4 or more, preferably 6 or more, preferably 8 or more, preferably 10 or more, preferably 13 or more, preferably 16 more, preferably 21 or more amino acids joined covalently by peptide bonds. The term "protein" refers large peptides, preferably to peptides with at least 160 amino acids, but in general the terms "peptides" and "proteins" are synonyms and are used in this application as synonyms. The terms "peptide" and "protein" according to the invention include substances containing not only amino acids, but also substances also containing non-amino acid constituents and include substances containing only peptide bonds as well as substances also containing other bonds, e.g. ester, thioether or disulfide bonds.

Peptides and variants thereof having less than about 100 amino acids, and generally less than about 50 amino acids, may be generated by synthetic means, using techniques well known to those of ordinary skill in the art. For example, such peptides may be synthesized using any of the commercially available solid-phase techniques, such as the Merrifield solid-phase synthesis method, where amino acids are sequentially added to a growing amino acid chain. See Merrifield, 1963, J. Am. Chem. Soc. 85, 2149-2146. Equipment for automated synthesis of polypeptides is commercially available from suppliers such as Perkin Elmer/Applied BioSystems Division (Foster City, Calif.), and may be operated according to the manufacturer's instructions.

The peptides and variants described herein can be obtained, for instance, by designing peptides or proteins which include a protease recognition sequence and thus, are substrates of said protease.

Within certain specific embodiments, a peptide or protein may be a fusion peptide or fusion protein that comprises multiple sequences as described herein, or that comprises at least one sequence as described herein and an unrelated sequence. A fusion partner may, for example, assist in expressing the peptide or protein (an expression enhancer) at higher yields than the native recombinant peptide. Other fusion partners may be selected so as to increase the solubility of the peptide or protein or to enable the peptide or protein to be targeted to desired intracellular compartments. Still further fusion partners include affinity tags, which facilitate purification and/or detection of the peptide or protein.

Fusion peptides or fusion proteins may generally be prepared using standard techniques, including chemical conjugation. Preferably, a fusion peptide or fusion protein is expressed as a recombinant protein, allowing the production of increased levels, relative to a non-fused peptide or protein, in an expression system. Briefly, DNA sequences encoding the peptide or protein components may be assembled separately, and ligated into an appropriate expression vector. The 3' end of the DNA sequence encoding one peptide or protein component is ligated, with or without a DNA sequence encoding a peptide linker, to the 5' end of a DNA sequence encoding the second peptide or protein component so that the reading frames of the sequences are in phase. This permits translation into a single fusion peptide or fusion protein that retains the biological activity of both component peptides or proteins.

A peptide linker sequence may be employed to separate the first and second peptide or protein components by a distance sufficient to ensure that each peptide or protein folds into its secondary and tertiary structures. Such a peptide linker sequence is incorporated into the fusion peptide or fusion protein using standard techniques well known in the art. Suitable peptide linker sequences may be chosen based on the following factors: (1) their ability to adopt a flexible extended conformation; (2) their inability to adopt a secondary structure that could interact with functional epitopes on the first and second peptides or proteins; and (3) the lack of hydrophobic or charged residues that might react with the peptide or protein functional epitopes. The linker sequence may generally be from 1 to about 50 amino acids in length. Linker sequences are not required when the first and second peptides or proteins have non-essential N-terminal amino acid regions that can be used to separate the functional domains and prevent steric interference.

### Modifications of peptides and proteins

Modifications of peptides or proteins described herein may comprise modifications due to posttranslational modifications, chemical modifications, enzymatical modifications and modifications due to other mechanisms. Examples of possible modifications include but are not limited to: glycosylation, phosphorylation, sulphatation, pyroglutamate modification, cystein-disulfide bridges, methylation, acetylation, acylation, farnesylation, formylation, geranylgeranylation, biotinylation, stearoylation, palmitylation, lipolyation, C-mannosylation, myristoylation, amidation, deamidation, methylation, demethylation, carboxylation, hydroxylation, iodination, oxidation, pegylation, prenylation, ADP-ribosylation, addition of lipids, of phosphatidylinositol, of glycosylphosphatidylinositol (GPI)-anchor, of pyridoxal phosphate, modification of cysteine residues resulting in carboxyamidomethylcysteine, resulting in carboxymethylcysteine, or resulting in pyridylethylcysteine, modification of lysine residues resulting in liponic acid, modification of glutamic acid resulting in pyroglutamic acid, etc.

Modifications of peptides or proteins described herein may comprise unusual amino acids, chemically or enzymatically modified amino acids etc. including, but not limited to: alpha amino butyric acid, beta amino butyric acid, beta amino iso-butyric acid, beta alanine, gamma butyric acid, alpha amino adipic acid, 4-amino benzoic acid, amino ethyl cysteine, alpha amino penicillanic acid, allysine, 4-carboxy glutamic acid, cystathionine, carboxy glutamic acid, carboxy amido methyl cysteine, carboxy methyl cysteine, cysteine acid, citrulline, dehydroalanine, di-amino butyric acid, dehydro amino-2-butyric acid, ethionine, glycine-proline di-peptide, 4-hydroxyproline, hydroxylysine, hydroxyproline, homoserine, homo cysteine, histamine, iso-valine, lysinoalanine, lanthionine, norvaline, norleucine, ornithine, 2-pipiridine-carboxylic acid, pyroglutamic acid, pyrrolysine, proline-hydroxy proline di-peptide, sarcosine, 4-selenocysteine, syndesine, thioproline, etc. Further examples can be found in databases such as the "Delta Mass" database searchable at the website of the ABRF, the "Association of Biomolecular Resource Facilities": http://www.abrf.org/index.cfm/dm.home?AvgMass=all.

### Peptidomimetics of peptides and proteins

Peptides and proteins described herein can be prepared or synthesized to represent partially or completely peptidomimetics. This allows to design peptides with more defined properties. For example D-amino acids could be used to prevent proteolysis. "Peptidomimetics" according to the invention are structures, which mimic the function of amino acids or amino acid sequences. "Peptidomimetics" or "mimetics of peptides" often have additional properties such as increased resistance to proteolysis. Peptidomimetics can replace some or all peptide bonds by other kinds of covalent bonds which can connect amino acids. Peptidomimetics among others can comprise modifications of amino acids such as alpha-C alkylation, alpha-N alkylation, etc, dipeptide analogues (two amino acid side chains which are connected by covalent bonds) or modifications of the peptide backbone, especially change from L- to D-amino acids, inverse N- to C-sequence, amide bond isosteres, etc. Further examples of peptidomimetics are spiegelmers® (NOXXON Parma AG, Berlin, Germany) or beta amino acids.

### Nucleic acids, complementary and degenerated nucleic acids and derivatives

With nucleic acids or nucleic acid sequences according to the invention are meant all kinds of deoxyribonucleic acids (DNA) and ribonucleic acids (RNA) or combinations thereof regardless if these nucleic acid molecules are single or double-stranded, linear, circular or branched. Preferably the nucleic acid molecules are purified nucleic acid molecules which means that the preparation contains at least 50 %, preferably 60 %, 70 %, 80 %, 90 %, 95 %, 99 %, preferably more than 99 % of a certain nucleic acid molecule or of a certain mixture of more than one certain nucleic acid molecule. Examples of nucleic acid molecules are genomic DNA, cDNA (complementary DNA), mRNA (messenger RNA), recombinantly produced nucleic acid molecules, chemically synthesized nucleic acid molecules or enzymatically generated nucleic acid molecules for example by use of polymerase chain reaction (PCR) or nucleic acid molecules purified from natural sources such as prokaryotic or eukaryotic cells, viruses, tissues, organs or whole organisms such as bacteria, yeast, insects, worms, etc. using methods known in the art. Furthermore nucleic acid molecules according to the invention can be partially or completely represent derivatives of nucleic acids which derivatives comprise nucleotides or nucleotide-like molecules not found in nature such as phosphorothioates, peptide nucleic acids (PNAs), N3',P5'-phosphoramidates, morpholino phosphoroamidates, 2'-*O*-methoxyethyl nucleic acids, 2'-fluoro-nucleic acids, arabino-nucleic acids, locked nucleic acids (LNA, ribonucleotides containing a methylene bridge that connects the 2'-oxygen of ribose with the 4'-carbon), etc.

Nucleic acids according to the invention furthermore comprise antisense nucleic acids, ribozymes, triplex-forming nucleic acids, RNAi-nucleic acids, nucleic acids suitable as primer for polymerase chain reactions, suitable for in situ hybridization, suitable for hybridization experiments such as Northern or Southern-blots or suitable for nucleic acid chip experiments, nucleic acids suitable as vectors or viral vectors, preferably for expression of peptides or proteins comprising at least one peptide or protein selected from the group consisting of Peptides / Proteins No. 1 to 106 of Table 1 or for expression of antisense nucleic acids directed to suppress expression of said peptides or proteins. The nucleic acids preferably have a sequence length which corresponds to the full lengths of said peptides or proteins, preferably at least 60%, preferably at least 80% of said full length of said peptides or proteins, depending on the intended use. If the nucleic acid represents an expression vector, the length of the nucleic acid molecule can be much longer and up to several thousands of nucleotides as many viruses such as for example bacculoviruses have large genomes resulting in very large vectors. Antisense and triplex-forming nucleic acid molecules preferably have a sequence length of 6 to 50, preferably 10 to 30, 15 to 30, 20 to 30 nucleotides. Ribozymes preferably have sequences hybridizing to the target RNA, which hybridizing sequences have similar length as corresponding antisense nucleic acids and in addition comprise a catalytic ribozyme sequence as known in the art. Nucleic acids for use as primer in polymerase chain reactions preferably have a length of 10 to 60, preferably 15 to 60, 15 to 50, 15 to 40, 15 to 30 nucleotides.

With complementary nucleic acid sequences are meant nucleic acid sequences which hybridize to each other to form a duplex or triplex nucleic acid molecule. The hybridization preferably takes place under stringent experimental conditions which ensure that the hybridization is sequence specific and not random. Stringent hybridizations conditions for nucleic acids are known in the art, and are published for example in Molecular Cloning: A Laboratory Manual, J. Sambrook et al, Cold Spring Harbor, New York, 1998 or in Current Protocols in Molecular Biology, F. M. Asibeö et al, John Wiley & Sons, Inc., New York. An example for stringent conditions for hybridization conditions is the hybridization at 65°C in a buffer containing 3.5x SSC, 0.02 % Vicoll, 0.02 % Polyvinylpyrrolidon, 0.02 % bovine serum albumin, 2.5 mM NaH₂PO₄ (pH 7), 0.5 % SDS (sodium dodecyl sulphate), 2 mM EDTA (Ethylenediaminetetraacetic acid). SSC represents a buffer containing 0.15 M sodium chloride and 0,15 M sodium citrate at pH 7. Subsequently to hybridization the membrane with the duplex nucleic acid sticking to it is washed at room temperature with 2x SSC and with 0.1 to 0.5x SSC with 0.1 % SDS at temperatures up to 68 °C. Other protocols for stringent experimental hybridization conditions are known in the art and are within the scope of the invention. A specific hybridization requires that at least 40 %, preferably 50 %, 60 %, 70 %, 80 %, 90 %, 95 %, 99 % of the parts of the nucleic acid sequences hybridizing to each other are complementary to each other. It is not necessary that the complete nucleic acid sequences hybridize to each other, as long as the duplex nucleic acid structure is stable under stringent experimental conditions.

With degenerated nucleic acid sequences is meant that a distinct amino acid sequence can be encoded for by various nucleic acid sequences, as most amino acids are encoded by at least two different codons. For this reason identical amino acid sequence can be encoded by very different sequences of nucleotides.

Nucleic acids can be labelled basically with the same functional structures as those structures used for labelling of peptides and proteins. Preferably nucleic acids are labelled with radioactive phosphorus, with small organic molecules such as digoxigenin, biotin or others, or with fluorophores or with label-nucleic acid sequences, which can be used to detect the labelled nucleic acid for example by using PCR-primers, northern blotting probes, or DNA-chips specific for said label-nucleic acid sequences.

### Methods to analyse peptides and proteins

Generally all methods suitable to detect and analyse peptides and proteins can be used in the methods of the invention and can be used to determine the presence, absence or quantity of the peptides and proteins described herein, in particular peptides or proteins comprising at least one peptide or protein selected from the group consisting of Peptides/Proteins 1 to 106 of Table 1, and thus can be used to indirectly detect the presence, absence, quantity and/or activity of a protease belonging to the POP-family of proteases. Preferably mass spectrometric methods, protein chip assays, immunological and molecular biology methods can be used for this purpose.

Suitable mass spectrometric methods among others are matrix assisted laser desorption ionisation (MALDI), continuous or pulsed electrospray ionization (ESI) and related methods such as ionspray or thermospray or massive cluster impact (MCI). The ion sources can be matched with detection formats including linear or non-linear reflection time-of-flight (TOF), single or multiple quadrupole, single or multiple magnetic sector, fourier transform ion cyclotron resonance (FTICR), ion trap, and combinations thereof, e.g. ion-trap/time-of-flight. For ionisation, numerous matrix/wavelength combinations (MALDI) or solvent combinations (ESI) can be used. Other mass spectrometric methods suitable are for example fast atom bombardment (FAB) mass spectrometry, Surface Enhanced Laser Desorption/Ionisation (SELDI) mass spectrometry, isotope coded affinity tag (ICAT) mass spectrometry, or affinity mass spectrometric methods.

Furthermore suitable immunologic methods among others are enzyme linked immuno assays (ELISA), sandwich, direct, indirect, or competitive ELISA assays, enzyme-linked immunospot assays (ELISPOT), radio immuno assays (RIA), flow cytometry assays (FACS = fluorescence activated cell sorting), immunohistochemistry, Western blot, fluorescence resonance energy transfer (FRET) assays, protein-chip assays using for example antibodies, antibody fragments, receptors, ligands, or other binding agents specific for the peptides or proteins described herein.

Among others Northern or Southern blot hybridization, nucleic acid dot- or slot-blot hybridization, in situ hybridization, nucleic acid chip assays (for example using RNA, DNA or other nucleic acids to specifically capture nucleic acid of interest), polymerase chain reaction (PCR), reverse transcriptase PCR (RT-PCR), real time PCR (taq-man PCR) can be used as molecular biology methods to detect the nucleic acids described herein.

Further methods such as nuclear magnetic resonance (NMR), fluorometry, colorimetry, radiometry, luminometry, or other spectrometric methods, liquid chromatography, capillary chromatography, thin-layer chromatography, plasmon resonance (BIACORE), one- or two-dimensional gel electrophoresis, etc. can be used to detect the peptides, proteins or nucleic acids described herein.

### Examples

The following examples are intended to illustrate the invention, however they are not meant to limit the scope of the invention in any way.

### Example 1: In vivo DPP4 inhibition in rats

The DPP4 inhibitor AB192 (bis(4-acetamidophenyl)-1-(S)-prolylpyrrolidine-2-(R,S)-phosphonate) (J Med Chem, 1999, 42:1041-52) was kindly provided by Ingrid De Meester (University of Antwerp, Campus Drie Eiken, Department of Pharmaceutical Sciences, Universiteitsplein 1, B-2610 Wilrijk-Antwerpen). AB192, an irreversible inhibitor of DPP4, was used at 0.32, 1.00 and 3.2 mg/kg body weight. The control inhibitor (Contr.ln.) was used at 0.3, 1.0 and 3.0 mg/kg body weight. Eight week old Wistar rats (315-320g body weight) received either of the inhibitors (8 animals for each inhibitor concentration of each inhibitor) or were treated with vehicle (50 mM sodium phosphate buffer; pH 7.4) (4 animals). The rats received the inhibitors or vehicle by subcutaneous injection into the fat tissue of the neck using a 27-G needle. Blood samples (100 µl) for DPP4 activity measurements were drawn from the tail vein before injection and at the end of the experiment 1 h after injection of the DPP4 inhibitors. At this point, the rats were anaesthetised using a mixture of ketamin (9 mg/100 g body weight) and rompun (0,2 mg/100 g body weight). Additionally, rats received a short burst of ether for quick anaesthesia. The thorax was opened immediately and 4 to 6 ml blood taken from the right ventricle into ethylene-diamine-tetra-acetic acid (EDTA) laminated syringes and transferred into a 15 ml tube filled with 2.5 mg/ml EDTA final concentration.

### Example 2: Measurement of DPP4 activity

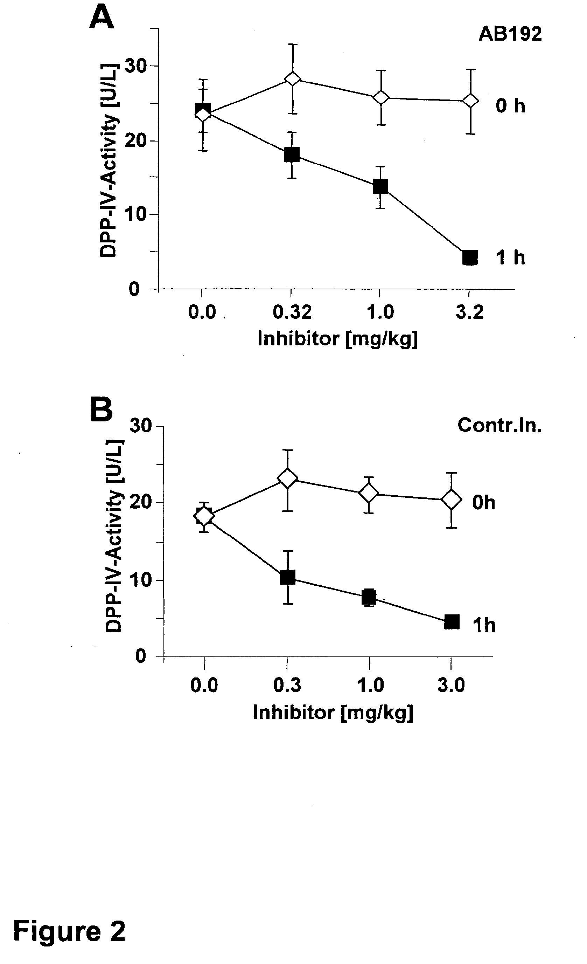

Blood samples taken from the tail vein were immediately centrifuged (2000 x g; 10 min at room temperature) and plasma stored at -20°C. At the day of the measurement 10 µl of each sample was pipetted in duplicates directly in a 96-well microtiter plate placed on ice. After all samples and standards (0-0.5 mU/ml purified DPP4 enzyme from porcine kidney; Sigma-Aldrich Chemie GmbH, Munich, Germany) were added, the plate was placed on a 37°C heating block and 40 µl freshly prepared substrate solution (500 µM G-P-4 nitroanilide diluted in 0.4 mM HEPES buffer) added to each well. The 96-well plate was read immediately using a Tecan Genios microplate reader (Tecan Deutschland GmbH, Crailsheim, Germany) and measurements were taken at 37°C over a period of time of 1 h at a wavelength of 405 nm. From the measured values the blank signal (plasma + HEPES buffer) was subtracted and data calculated as Units/litre (U/I). The results of the measurements of the DPP4 activity measured in plasma of rats treated with two different inhibitors (AB192 and Contr. In.) at various concentrations and in plasma of control rats (treated with vehicle) is shown in Figure 2. Both inhibitors tested inhibited the activity of DPP4 in vivo in rats in a dose dependent manner.

### Example 3: Rat plasma preparation for peptide display

Rat blood samples were consecutively centrifuged at room temperature for 10 min at 2000 x g and for 15 min at 2500 x g. The resulting platelet poor plasma was stored at -80°C until further analyzed. For reduction of the protein load of the plasma, the plasma sample was diluted 1:4 with 8 M guanidinium chloride solution at room temperature and for quality control purposes a peptide standard mix was added resulting in a final concentration of 300 to 800 pM of each individual standard peptide added. The peptides added were: Substance P, Somatostatin-14, Neurotensin, Renin Substrate (porcine); Adrenocorticotropic Hormone (ACTH)(1-17), Big Endothelin-1(19-38), ACTH(18-39), ß-Endorphin(6-31), Adrenocorticotropic Hormone(1-24), Calcitonin (human), Insulin B chain oxide (bovine), ACTH(7-38), Pro34-Neuropeptide Y (porcine) and growth hormone-releasing factor (GRF)(1-44). All Peptides or proteins represent the human sequence, if not indicated to the contrary and the numbers in brackets following some of the peptides indicate the amino acid positions of the complete sequence including said peptide. Prior to use, the ultrafiltration devices are rinsed with distilled water. Ultrafiltration was done using Amicon Ultra filter devices with a molecular weight cut-off of 50 kDa (Millipore, Bedford, USA), which were centrifuged according to the manufacturers instruction at 4000 x g for 60 min at room temperature in a swinging bucket rotor. Finally, the pH of the filtrate was adjusted to pH 2 to 3 using concentrated HCl (30% v/v). If the sample (filtrate) was not directly further processed, it was stored at -80 °C. A 750 µl-equivalent of plasma was used per chromatographic run.

### Example 4: Liquid chromatography of the samples

The separation of peptides and/or proteins was done using a Source 5RPC, 4,6 x 150 mm reverse phase chromatography column (Amersham Biosciences Europe GmbH, Freiburg, Germany). Buffer A was 0.06 % (v/v) (volume per volume) trifluoroacetic acid (TFA) in distilled water and buffer B was 0.05 % (v/v) TFA, 80 % (v/v) acetonitrile in distilled water.

The chromatography took place at 33 °C using a HP 1100 HPLC with a micro flow cell both supplied by Agilent Technologies, Böblingen, Germany. The samples were diluted to 5.5 ml using a 0.1 % (v/v) stock solution of trifluoroacetic acid. Prior to chromatography, the samples were centrifuged at 4 °C and 15 000 x g for 10 minutes and finally 5 ml of sample were loaded onto the chromatography column representing an equivalent of 750 µl plasma. Buffers A and B were mixed in various ratios during chromatography and in the following only the amount in percentage of buffer B is stated. The amount in percentage of buffer A is 100 % minus % buffer B (v/v). The chromatography conditions were as follows:
5 % buffer B for 1 min,
a linear gradient from 5 to 50 % buffer B during the next 44 min.
a linear gradient from 50 to 100 % buffer B during the next 4 min.
100 % buffer B for the next 4 min.
The flow rate was 500 µl/min and 96 fractions each containing 0.25 ml were collected during the gradients from 5 to 100 % buffer B.

### Example 5: Mass spectrometry of the samples

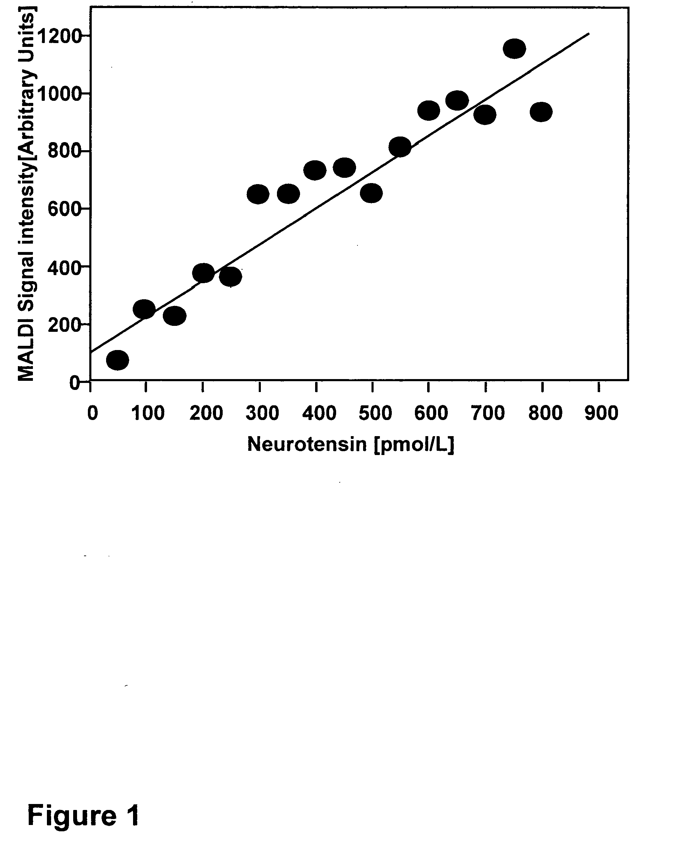

For mass spectrometric analysis, typical positive ion spectra of peptides were measured using a MALDI-TOF (matrix-assisted laser desorption ionization time of flight) mass spectrometer. For MALDI sample preparation alpha-cyano-4-hydroxycinnamic acid was used as matrix and 6-desoxy-I-galactose was used as co-matrix, dissolved in acetonitrile containing 0.1 % TFA. A lyophilized equivalent obtained by reverse phase chromatography corresponding to 7.5 µl plasma spotted onto the mass spectrometric carrier plate was used to measure the peptides and/or proteins. The fractionated, lyophilized sample was dissolved in 15 µl of a matrix solution. This matrix solution contained 10 g/L α-cyano-4-hydroxycinnamic acid and 10 g/L L(-)fucose dissolved in a solvent mixture consisting of acetonitrile, water, trifluoroacetic acid (TFA) and acetone in a ratio of 49:49:1:1 by volume (v/v). 0.3 µl of this sample solution was transferred to a MALDI carrier plate, and the dried sample was analyzed in a Voyager-DE STR MALDI mass spectrometer from PerSeptive Biosystems. The measurement took place in linear mode with delayed extraction™. A MALDI-TOF mass spectrometer can be employed to quantify peptides such as, for example, the peptides and proteins described herein if these peptides and proteins are present in a concentration which is within the dynamic measurement range of the mass spectrometer, thus avoiding detector saturation. The ability of MALDI mass spectrometry to quantitate individual peptide and/or protein mass signals was confirmed by adding known concentrations of 50 to 800 pmol peptides, such as neurotensin, into complex biological samples such as plasma samples. Subsequently these samples were separated by reversed phase chromatography and those fractions containing the added peptides were analyzed by mass spectrometry as described in the examples. There is a linear correlation between the MALDI mass spectrometry mass signal intensity of, for example, the neurotensin peptide and the amount of the neurotensin peptide added to the plasma sample, indicating that MALDI mass spectrometry is suitable to quantitatively determine peptides in complex samples such as plasma (Figure 1). The correlation of the mass signal of exemplary peptides of Table 1 and of the DPP4 activity measured in the same samples or of the amount of inhibitor applied to the experimental rats is shown in Figures 3 and 4 and in Table 1.

### Example 6: Peptide identification

Detection of differentially expressed peptides was achieved by calculation of subtractive peptide display maps and correlation analysis. Selected peptides, which appeared in peptide display maps of plasma from rats were analyzed by ESI-qTOF sequencing (PE Applied Biosystems, Framingham, USA). Peptide ions were selected in the mass spectrometer on the basis of their specific m/z (mass/charge) values in a manner known to the skilled worker. These selected ions were then fragmented by supplying collision energy (20 - 40 eV) with an collision gas, e.g. helium or nitrogen, and the resulting fragments of the peptides or proteins were detected in the mass spectrometer in an integrated analysis unit, and corresponding m/z values were determined (principle of tandem mass spectrometry). The fragmentation behaviour of peptides enables the unambiguous identification of the peptides. The mass-spectrometric analysis can be done for example by Quadrupol-TOF (time of flight) sequencing (QStar-Pulsar model) or ESI-qTOF sequencing (both from PE Applied Biosystems, Framingham, USA). The resulting peptide fragment spectra were detected using nanoSpray in the product ion scan mode (spray voltage 950 V, collision energy 20-40 eV). Up to 200 scans per sample were accumulated. Charge state deconvolution was performed using the Bayesian reconstruct tool of the BioAnalyst program package (PE Applied Biosystems), and deisotoping was achieved by means of the Voyager 5.1 software (PE Applied Biosystems). The mass spectra were saved in a MASCOT generic file format, and submitted to the MASCOT19 database search engine (Matrix Science, London, UK). Searched databases were SwissProt (Version 39.6, www.expasy.ch) and MSDB (Version 010721, EBI, Europe). In addition, this peptide sequencing method allows the identification of amino acid modifications such as phosphorylation, acetylation or hydroxylation.

### Example 7: Data analysis

Subsequently to fractionation as described in example 4, each fraction was individually analyzed by MALDI mass spectrometry as described in example 5 resulting in 96 mass spectra for each sample. These 96 mass spectra were electronically combined resulting in a so called peptide display. The x-axis of these peptide displays depicts the molecular mass, the y-axis depicts the fraction number and the colour intensity represents the mass spectrometric signal intensity. The data of peptide displays may be pre-processed by minimizing background noise, and outliers may be detected, e.g. visually or by means of principal component analysis (Pirouette 3.0, Infometrix Inc, WA, USA), and excluded from further analysis.
Peptide signals, whose concentration and thus, mass spectrometric signal intensity correlated with an endpoint such as the concentration of the applied inhibitor, were in focus of investigation: Various correlations were performed correlating the mass spectrometric signal intensities with meta data such as the concentration of the inhibitor used or with the change in DPP4 activity ("delta DPP4 activity" = "before/after change in DPP4 activity") determined in rat plasma after inhibitor administration to the experimental rats.

Mass spectrometric signals correlating with
A) the concentration of AB192 and with the concentration of the control inhibitor (Contr. In.) (n= 49, |r|>0.5)
B) the concentration of AB192, but not with the concentration of the control inhibitor (n= 29, |r|>0.59)
C) the concentration of the control inhibitor, but not with the concentration of AB192 (n= 27, |r|>0.61)
D) the "before/after change in DPP4 activity" in the presence of AB192 and correlating with the "before/after change in DPP4 activity" in the presence of the control inhibitor (n= 49, |r|>0.5)
E) the "before/after change in DPP4 activity" in the presence of AB192 but not with the "before/after change in DPP4 activity" in the presence of the control inhibitor (n= 29, |r|>0.59)
F) the "before/after change in DPP4 activity" in the presence of the control inhibitor but not with "before/after change in DPP4 activity" in the presence of AB192 (n= 31, |r|>0.61)
were searched. This correlation analysis for example can be performed by means of Spearman correlation, e.g. with R-package, version 2.0 (R Foundation for Statistical Computing, Vienna, Austria) or S-plus, version 6.0 (Insightful AG, Reinach, Switzerland), etc.

In order to exclusively obtain signals within the 95 % confidence interval, which depends on the number of samples analyzed, different threshold values of Spearman's correlation coefficients ( |r| ) were used (see A to F above) to determine the significant peptide coordinates. Furthermore, the threshold values of change of signal intensities of the control group (no inhibitor added) to the groups having received inhibitors, should be determined empirically, and was in this example 18 arbitrary units. Subsequently redundant signal coordinates were removed resulting in the remaining list of signal coordinates shown in Table 1. Redundant signals are, for example, signal coordinates with the same m/z values in directly adjacent fractions, which most likely represent the same peptide which by chance eluted in two or more adjacent fractions, or m/z values representing multiples of a m/z value (e.g. 2050 and 4100) and which are present in the same or directly adjacent fractions (most likely representing multiple charged versions of an ion of the same molecule). Each signal coordinate is only listed once in Table 1. If the same signal coordinate (Frac. and m/z) was found in more than one of the groups A to F, this fact is indicated in Table 1.

In addition networks of peptides were calculated. This allows, for example, to identify panels of peptides, which enable to better reflect the biological impact of the inhibitors (Figure 5 /Table 2). Combining two peptides in these panels increased the reliability of the prediction (e.g. concentration of inhibitor, "before/after change in DPP4 activity") possible by evaluating these peptide panels. These panels were determined by comprehensively combining peptide signals to pairs of peptide signals. In a "linear model", the signal intensities of the peptide pairs were treated as independent variables, and the endpoint DPP4 concentration (or change in DPP4 activity) was treated as the dependent variable. The "linear model" was then calculated (e.g. with R-package, version 2.00 (R Foundation for Statistical Computing, Vienna, Austria) or S-plus, version 6.0 (Insightful AG, Reinach, Switzerland), etc.). Those models with pairs of peptide signals, which resulted in a better prediction of the endpoint compared to separate single peptide signals, as indicated by an increased correlation coefficient in row "Panel 1 +2", are putative panels of peptides.

In addition to panels of peptides, surrogate networks of peptides can also be predicted, which peptides can replace each other, i.e. the peptides are surrogates of each other. Using this method it is, for example, possible to identify peptides which have the same diagnostic value. Therefore one can avoid to measure more than one of these peptides, since measuring additional members of the same surrogate network does not give any additional diagnostic information. In addition, it is possible to choose those peptides of a surrogate network, which can be measured with the least effort. Both strategies can be combined to save time, resources, valuable or limited patient samples, etc. For determining these surrogate networks, and peptide panels methods such as the methods described in PCT/EP 2005/000090 can be used.

**Table 1: Changes of mass spectrometric signals of peptides or proteins depending on inhibitors (AB and/or Contr. In.)**

| Peptide / Protein No. | Frac. | m/z | Sig- nal | Correlation of mass spectrometric signal with | | | | | | Sequence |
|---|---|---|---|---|---|---|---|---|---|---|
| | | | | concentration of inhibitor | | | DPP4 inhibition by | | | |
| | | | | AB & Contr. In. | AB | Contr. In. | AB & Contr. In. | AB | Contr. In. | |
| | | | | A | B | C | D | E | F | |
| 1 | 23 | 1737 | 647 | --- | --- | --- | --- | --- | pos. | SEQ ID NO: 1 Col a1 (I) rat 425-443 |
| 2 | 42 | 2323 | 610 | pos. | --- | --- | --- | --- | --- | SEQ ID NO: 2 Col a1(I) rat 318-342 |
| 3 | 30 | 1369 | 392 | --- | --- | pos. | --- | --- | --- | SEQ ID NO: 3 Col a1 (II) rat 100-114 |
| 4 | 30 | 1446 | 273 | --- | --- | pos. | --- | --- | --- | SEQ ID NO: 4 Col a2(I) rat 1097-1113 |
| 5 | 49 | 2607 | 237 | --- | --- | --- | --- | pos. | --- | SEQ ID NO: 5 FibA rat 333-358 |
| 6 | 30 | 3335 | 176 | --- | --- | pos. | --- | --- | --- | |
| 7 | 31 | 3318 | 136 | --- | --- | pos. | --- | --- | --- | |
| 8 | 52 | 1363 | 89 | --- | pos. | --- | --- | --- | --- | |
| 9 | 58 | 2675 | 62 | --- | --- | --- | pos. | --- | --- | |
| 10 | 30 | 3351 | 59 | --- | --- | pos. | --- | --- | --- | *SEQ ID NO: 6 Col a1(III) mouse 275-310 |
| 11 | 40 | 2244 | 58 | --- | pos. | --- | --- | pos. | --- | |
| 12 | 25 | 1849 | 55 | --- | --- | --- | --- | --- | pos. | |
| 13 | 42 | 3187 | 54 | --- | pos. | --- | --- | pos. | --- | |
| 14 | 25 | 1721 | 49 | --- | --- | pos. | --- | --- | pos. | |
| 15 | 30 | 3469 | 43 | --- | --- | pos. | --- | --- | --- | |
| ** 16 | 51 | 2676 | 43 | --- | --- | pos. | --- | --- | pos. | |
| 17 | 56 | 2744 | 38 | --- | pos. | --- | --- | --- | --- | |
| 18 | 56 | 5489 | 38 | --- | pos. | --- | --- | pos. | --- | |
| 19 | 25 | 1436 | 36 | --- | --- | --- | pos. | --- | --- | |
| 20 | 60 | 2751 | 23 | --- | pos. | --- | --- | pos. | --- | |
| 21 | 28 | 2940 | 544 | --- | --- | neg. | --- | --- | --- | |
| 22 | 49 | 2336 | 470 | --- | --- | neg. --- | | --- | neg. | |
| 23 | 26 | 3267 | 458 | --- | --- | neg. | --- | --- | neg. | |
| 24 | 79 | 2711 | 452 | --- | --- | --- | neg. | --- | --- | |
| 25 | 25 | 3267 | 409 | --- | --- | --- | --- | --- | neg. | |
| 26 | 73 | 3868 | 364 | --- | --- | --- | --- | neg. | --- | |
| 27 | 25 | 3210 | 297 | --- | --- | --- | --- | --- | neg. | |
| 28 | 25 | 2094 | 281 | --- | --- | --- | neg. | --- | --- | |
| 29 | 30 | 2589 | 261 | --- | --- | --- | neg. | --- | --- | |
| 30 | 74 | 3867 | 241 | --- | --- | --- | --- | neg. | --- | |
| 31 | 31 | 3148 | 234 | neg. | --- | --- | neg. | --- | --- | |
| 32 | 39 | 2039 | 193 | --- | --- | neg. | --- | -- | neg. | |
| 33 | 38 | 4086 | 191 | --- | --- | --- | --- | --- | neg. | |
| 34 | 76 | 2711 | 182 | --- | --- | --- | neg. | --- | --- | |
| 35 | 29 | 2603 | 174 | --- | --- | neg. | --- | --- | neg. | |
| 36 | 28 | 3680 | 172 | --- | --- | neg. | --- | --- | --- | |
| 37 | 27 | 3251 | 165 | --- | --- | neg. | neg. | --- | --- | |
| 38 | 77 | 2711 | 164 | --- | --- | --- | --- | neg. | --- | |
| 39 | 68 | 1562 | 161 | --- | neg. | --- | --- | --- | --- | |
| 40 | 59 | 2475 | 146 | --- | --- | neg. | --- | --- | --- | |
| 41 | 23 | 2650 | 142 | --- | --- | neg. | --- | --- | --- | |
| 42 | 68 | 3083 | 136 | --- | --- | --- | --- | neg. | --- | |
| 43 | 30 | 2532 | 132 | --- | --- | --- | --- | --- | neg. | |
| 44 | 37 | 1679 | 119 | neg. | --- | --- | neg. | --- | --- | |
| 45 | 21 | 2579 | 112 | --- | --- | --- | --- | --- | neg. | SEQ ID NO: 7 Col a1(III) rat 812-839 |
| 46 | 75 | 2711 | 104 | --- | --- | --- | --- | neg. | --- | |
| 47 | 23 | 1938 | 101 | --- | --- | neg. | --- | --- | --- | |
| 48 | 26 | 1633 | 100 | --- | --- | neg. | --- | --- | neg. | |
| 49 | 28 | 2547 | 92 | --- | --- | neg. | --- | --- | neg. | SEQ ID NO: 8 Col 1 a rat 805-832 |
| 50 | 25 | 3283 | 92 | --- | --- | --- | --- | --- | neg. | |
| 51 | 26 | 3210 | 92 | --- | --- | neg. | --- | --- | --- | |
| 52 | 73 | 1934 | 91 | --- | neg. | --- | --- | neg. | --- | |
| 53 | 25 | 2454 | 87 | --- | --- | neg. | --- | --- | neg. | |
| 54 | 38 | 3493 | 87 | --- | --- | neg. | --- | --- | --- | |
| 55 | 25 | 3358 | 85 | --- | --- | neg. | --- | --- | --- | |
| ** 56 | 39 | 2097 | 82 | --- | --- | neg. | --- | --- | neg. | |
| 57 | 38 | 2041 | 80 | --- | --- | neg. | --- | --- | --- | |
| 58 | 51 | 2654 | 75 | --- | --- | neg. | --- | --- | --- | |
| 59 | 28 | 3696 | 75 | --- | --- | neg. | --- | --- | --- | |
| 60 | 30 | 1583 | 74 | --- | --- | --- | neg. | --- | --- | |
| 61 | 26 | 2501 | 70 | --- | --- | neg. | --- | --- | --- | |
| 62 | 24 | 3000 | 70 | --- | --- | neg. | --- | --- | neg. | |
| 63 | 36 | 3410 | 67 | --- | --- | neg. | --- | --- | --- | |
| 64 | 27 | 3194 | 64 | --- | --- | neg. | --- | --- | --- | |
| 65 | 33 | 2938 | 63 | --- | --- | neg. | --- | --- | neg. | |
| 66 | 95 | 1634 | 61 | --- | --- | --- | --- | neg. | --- | |
| 67 | 77 | 5390 | 56 | --- | --- | --- | --- | neg. --- | | |
| 68 | 27 | 2337 | 56 | --- | --- | neg. | --- | --- | neg. | |
| 69 | 15 | 1510 | 55 | --- | --- | neg. | --- | --- | neg. | |
| 70 | 35 | 2049 | 55 | --- | --- | neg. | --- | --- | --- | |
| 71 | 26 | 2234 | 54 | --- | --- | neg. | --- | --- | neg. | |
| 72 | 28 | 4922 | 54 | --- | --- | neg. | --- | --- | neg. | |
| 73 | 28 | 2192 | 52 | --- | --- | neg. | --- | --- | --- | |
| 74 | 28 | 2220 | 52 | neg. | --- | -- | --- | --- | --- | |
| 75 | 20 | 1277 | 50 | --- | --- | --- | --- | --- | neg. | |
| 76 | 28 | 2563 | 49 | --- | --- | neg. | --- | --- | neg. | |
| 77 | 22 | 2283 | 49 | --- | --- | neg. | --- | --- | neg. | |
| 78 | 33 | 2239 | 49 | --- | --- | neg. | --- | --- | --- | |
| 79 | 67 | 2473 | 48 | --- | --- | --- | --- | neg. --- | | |
| 80 | 56 | 4359 | 47 | --- | --- | --- | --- | neg. | --- | SEQ ID NO: 9 Col a2(I) rat 545-590 |
| 81 | 32 | 3132 | 46 | --- | --- | --- | --- | --- | neg. | |
| 82 | 32 | 2829 | 46 | --- | --- | neg. | --- | --- | --- | |
| 83 | 35 | 4228 | 42 | neg. --- | | --- | --- | --- | --- | |
| 84 | 68 | 3120 | 42 | --- | neg. | --- | --- | neg. | --- | |
| 85 | 25 | 3226 | 42 | --- | --- | --- | --- | --- | neg. | |
| 86 | 16 | 1510 | 41 | --- | --- | neg. | --- | --- | neg. | |
| 87 | 35 | 4720 | 41 | --- | --- | --- | --- | --- | neg. | |
| 88 | 27 | 3359 | 40 | --- | --- | --- | neg. | --- | --- | |
| 89 | 38 | 4286 | 40 | --- | --- | neg. | neg. | --- | --- | |
| 90 | 27 | 3266 | 39 | --- | --- | neg. | --- | --- | neg. | |
| 91 | 58 | 2474 | 37 | --- | --- | --- | neg. | --- | --- | |
| 92 | 23 | 2855 | 37 | --- | --- | neg. | --- | --- | neg. | |
| 93 | 29 | 2220 | 37 | --- | --- | --- | --- | --- | neg. | |
| 94 | 34 | 5876 | 36 | --- | --- | --- | neg. | --- | --- | |
| 95 | 36 | 4719 | 36 | --- | --- | neg. | neg. | --- | --- | |
| 96 | 28 | 2579 | 35 | --- | --- | neg. | --- | --- | --- | |
| 97 | 27 | 1625 | 32 | --- | --- | neg. | --- | --- | --- | |
| 98 | 31 | 2814 | 30 | --- | --- | --- | -- | --- | neg. | |
| 99 | 36 | 5844 | 30 | --- | --- | neg. | neg. | --- | --- | |
| 100 | 31 | 4467 | 28 | --- | --- | --- | --- | --- | neg. | |
| 101 | 24 | 2632 | 27 | --- | --- | --- | --- | --- | neg. | |
| 102 | 26 | 3283 | 24 | --- | --- | --- | --- | --- | neg. | |
| 103 | 35 | 4356 | 24 | neg. | --- | --- | --- | --- | --- | |
| 104 | 35 | 4735 | 24 | neg. | --- | --- | --- | --- | --- | |
| 105 | 36 | 2332 | 19 | --- | --- | neg. | --- | --- | --- | |
| 106 | 52 | 2638 | 17 | --- | --- | neg. | --- | --- | neg. | |

| | | | | | | | | | | |
|---|---|---|---|---|---|---|---|---|---|---|
| * SEQ ID NO: 6 presumably represents a murine sequence (according to sequence database searches performed). However, as the sample used to identify this sequence was from rat, SEQ ID NO: 6 represents a rat sequence not present in the databases. This sequence seems to be conserved between mice and rats. ** Peptide / Protein 56 and 16 are combined to panel 62 (Table 2, Figure 5), to improve the correlation coefficient. Frac. = Fraction; m/z = mass/charge; Signal = mass spectrometric signal intensity in arbitrary units [au], AB = AB192 DPP4 inhibitor; Contr.In. = control inhibitor, A to F reference the different experimental groups as described in a previous chapter of this application | | | | | | | | | | |

With positive (pos.) correlation regarding the inhibitor concentration is meant that higher concentrations of inhibitor result in higher concentration of a peptide or protein.

With positive correlation regarding the before/after change in DPP4 inhibition is meant, that less DPP4 activity results in lower concentration of a peptide or protein. Peptide or protein numbers 1 to 20 of Table 1 are increased in their amount present in plasma of DPP4 inhibitor treated rats and supposedly represent substrates of DPP4 and/or DPP8, peptide or protein numbers 21 to 106 of Table 1 are decreased in their amount present in plasma of inhibitor-treated rats and supposedly represent products of DPP4 and/or DPP8. Peptides are sorted firstly by positively correlating (peptides 1-20) and negative correlating (peptides 21-106) peptides and secondly by their respective mass spectrometric signal intensity (row "Signal").

**Table 2: Panels of pairs of peptides or proteins of Table 1 improving the overall correlation to inhibitor concentration (AB and/or Contr.In.) or to inhibitor-mediated decreased DPP4 activity**

| Panel No. | Peptide/Protein 1 | | Peptide/Protein 2 | | Correlation coefficient of | | |
|---|---|---|---|---|---|---|---|
| | | | | | Pep./Prot. 1 | Pep./Prot. 2 | Panel 1+2 |
| | Frac. | m/z | Frac. | m/z | with concentration of AB and Contr. In. | | |
| 1 | 37 | 1679 | 35 | 4228 | -0,35 | -0,38 | 0,43 |
| 2 | 31 | 3148 | 35 | 4356 | -0,52 | -0,52 | 0,59 |
| | | | | | with concentration of AB | | |
| 3 | 73 | 1934 | 56 | 2744 | -0,44 | 0,48 | 0,66 |
| 4 | 68 | 3120 | 56 | 2744 | -0,48 | 0,48 | 0,61 |
| | | | | | with concentration of Contr.In. | | |
| 5 | 31 | 3318 | 51 | 2676 | 0,44 | 0,38 | 0,66 |
| 6 | 30 | 3335 | 51 | 2676 | 0,49 | 0,38 | 0,69 |
| 7 | 31 | 3318 | 27 | 3266 | 0,44 | -0,47 | 0,65 |
| 8 | 28 | 2940 | 51 | 2676 | -0,42 | 0,38 | 0,63 |
| 9 | 28 | 3696 | 51 | 2676 | -0,45 | 0,38 | 0,64 |
| 10 | 28 | 3680 | 51 | 2676 | -0,45 | 0,38 | 0,64 |
| 11 | 32 | 2829 | 51 | 2676 | -0,45 | 0,38 | 0,63 |
| 12 | 25 | 2454 | 51 | 2676 | -0,38 | 0,38 | 0,56 |
| 13 | 31 | 3318 | 33 | 2239 | 0,44 | -0,5 | 0,61 |
| 14 | 30 | 3335 | 27 | 3266 | 0,49 | -0,47 | 0,65 |
| 15 | 28 | 4922 | 30 | 3469 | -0,59 | 0,6 | 0,72 |
| 16 | 30 | 1369 | 30 | 3335 | 0,52 | 0,49 | 0,66 |
| 17 | 30 | 3335 | 33 | 2239 | 0,49 | -0,5 | 0,63 |
| 18 | 30 | 3351 | 51 | 2676 | 0,6 | 0,38 | 0,73 |
| 19 | 31 | 3318 | 27 | 1625 | 0,44 | -0,42 | 0,57 |
| 20 | 31 | 3318 | 25 | 3358 | 0,44 | -0,46 | 0,57 |
| 21 | 31 | 3318 | 27 | 3194 | 0,44 | -0,45 | 0,57 |
| 22 | 59 | 2475 | 51 | 2676 | -0,52 | 0,38 | 0,64 |
| 23 | 30 | 3335 | 36 | 2332 | 0,49 | -0,5 | 0,61 |
| 24 | 31 | 3318 | 32 | 2829 | 0,44 | -0,45 | 0,56 |
| 25 | 30 | 3351 | 30 | 3469 | 0,6 | 0,6 | 0,71 |
| 26 | 30 | 3351 | 28 | 4922 | 0,6 | -0,59 | 0,71 |
| 27 | 33 | 2938 | 30 | 3351 | -0,6 | 0,6 | 0,71 |
| 28 | 51 | 2654 | 51 | 2676 | -0,56 | 0,38 | 0,67 |
| 29 | 31 | 3318 | 23 | 1938 | 0,44 | -0,41 | 0,55 |
| 30 | 31 | 3318 | 27 | 2337 | 0,44 | -0,43 | 0,55 |
| 31 | 36 | 3410 | 51 | 2676 | -0,39 | 0,38 | 0,51 |
| 32 | 27 | 3251 | 16 | 1510 | -0,55 | -0,48 | 0,65 |
| 33 | 30 | 3335 | 16 | 1510 | 0,49 | -0,48 | 0,59 |
| 34 | 30 | 3335 | 27 | 1625 | 0,49 | -0,42 | 0,59 |
| 35 | 30 | 3335 | 32 | 2829 | 0,49 | -0,45 | 0,59 |
| 36 | 28 | 3680 | 31 | 3318 | -0,45 | 0,44 | 0,56 |
| 37 | 31 | 3318 | 25 | 2454 | 0,44 | -0,38 | 0,54 |
| | | | | | with DPP4 activity in presence of AB and Contr.In. | | |
| 38 | 27 | 3251 | 27 | 3359 | -0,28 | -0,44 | 0,55 |
| | | | | | with DPP4 activity in presence of AB | | |
| 39 | 68 | 3083 | 60 | 2751 | -0,38 | 0,77 | 0,84 |
| 40 | 95 | 1634 | 60 | 2751 | -0,4 | 0,77 | 0,84 |
| 41 | 56 | 4359 | 56 | 5489 | -0,4 | 0,67 | 0,81 |
| 42 | 95 | 1634 | 68 | 3120 | -0,4 | -0,45 | 0,6 |
| 43 | 56 | 4359 | 68 | 3120 | -0,4 | -0,45 | 0,59 |
| 44 | 77 | 2711 | 95 | 1634 | -0,38 | -0,4 | 0,54 |
| 45 | 73 | 1934 | 56 | 4359 | -0,38 | -0,4 | 0,54 |
| 46 | 73 | 1934 | 95 | 1634 | -0,38 | -0,4 | 0,52 |
| 47 | 42 | 3187 | 60 | 2751 | 0,74 | 0,77 | 0,87 |
| 48 | 74 | 3867 | 56 | 4359 | -0,45 | -0,4 | 0,57 |
| 49 | 73 | 3868 | 56 | 4359 | -0,42 | -0,4 | 0,54 |
| 50 | 75 | 2711 | 67 | 2473 | -0,38 | -0,39 | 0,49 |
| 51 | 68 | 3083 | 75 | 2711 | -0,38 | -0,38 | 0,49 |
| | | | | | with DPP4 activity in presence of Contr. In. | | |
| 52 | 25 | 2454 | 51 | 2676 | -0,4 | 0,55 | 0,69 |
| 53 | 21 | 2579 | 51 | 2676 | -0,43 | 0,55 | 0,71 |
| 54 | 26 | 1633 | 51 | 2676 | -0,43 | 0,55 | 0,69 |
| 55 | 28 | 4922 | 51 | 2676 | -0,4 | 0,55 | 0,64 |
| 56 | 25 | 3267 | 39 | 2039 | -0,47 | -0,59 | 0,68 |
| 57 | 26 | 3267 | 51 | 2676 | -0,46 | 0,55 | 0,66 |
| 58 | 26 | 2234 | 51 | 2676 | -0,48 | 0,55 | 0,65 |
| 59 | 25 | 3267 | 16 | 1510 | -0,47 | -0,51 | 0,63 |
| 60 | 25 | 3267 | 21 | 2579 | -0,47 | -0,43 | 0,63 |
| 61 | 39 | 2039 | 38 | 4086 | -0,59 | -0,41 | 0,73 |
| *62 | 39 | 2097 | 51 | 2676 | -0,58 | 0,55 | 0,73 |
| 63 | 51 | 2676 | 24 | 2632 | 0,55 | -0,46 | 0,7 |
| 64 | 15 | 1510 | 51 | 2676 | -0,51 | 0,55 | 0,66 |
| 65 | 16 | 1510 | 31 | 4467 | -0,51 | -0,54 | 0,65 |
| 66 | 39 | 2039 | 51 | 2676 | -0,59 | 0,55 | 0,72 |
| 67 | 30 | 2532 | 51 | 2676 | -0,55 | 0,55 | 0,69 |
| 68 | 49 | 2336 | 51 | 2676 | -0,52 | 0,55 | 0,65 |
| 69 | 25 | 1721 | 51 | 2676 | 0,51 | 0,55 | 0,65 |
| 70 | 32 | 3132 | 51 | 2676 | -0,55 | 0,55 | 0,68 |
| 71 | 51 | 2676 | 27 | 3266 | 0,55 | -0,49 | 0,67 |
| 72 | 29 | 2603 | 51 | 2676 | -0,53 | 0,55 | 0,66 |
| 73 | 16 | 1510 | 52 | 2638 | -0,51 | -0,49 | 0,64 |
| 74 | 51 | 2676 | 26 | 3283 | 0,55 | -0,48 | 0,67 |
| 75 | 25 | 3283 | 16 | 1510 | -0,54 | -0,51 | 0,66 |
| 76 | 25 | 3267 | 26 | 1633 | -0,47 | -0,43 | 0,59 |
| 77 | 51 | 2676 | 29 | 2220 | 0,55 | -0,53 | 0,66 |
| 78 | 39 | 2039 | 28 | 2547 | -0,59 | -0,48 | 0,68 |

| | | | | | | | |
|---|---|---|---|---|---|---|---|
| Frac. = Fraction; m/z = mass/charge; AB = AB192 inhibitor; Contr.In. = control inhibitor, Pep./Prot. = Peptide / Protein = Peptide or Protein, Panel 1+2 = Panel made by use of Peptide/Protein 1 and Peptide/Protein 2 * This peptide panel is shown in Figure 5 and results in an improvement of the correlation coefficient for Peptide/Protein no. 56 and 16 from Table 1 from r = -0.58 and r = 0.55 for the individual peptides/proteins, respectively, to r = 0.73 for the peptide panel combining both peptides/proteins. | | | | | | | |

## Claims

1. A method of determining the activity and/or amount of a protease belonging to the prolyl oligopeptidase family (POP-family) in a sample comprising the steps of allowing a protease belonging to the POP-family present in the sample to contact a substrate for a protease belonging to the POP-family and determining the amount of at least one peptide or protein comprising at least one peptide or protein selected from the group consisting of Peptides/Proteins 1 to 106 of Table 1.

2. The method of claim 1 further comprising the step of comparing the amount of the at least one peptide or protein comprising at least one peptide or protein selected from the group consisting of Peptides/Proteins 1 to 106 of Table 1 determined with a reference value.

3. The method of claim 2 wherein the reference value is derived from the determination of the amount of said at least one peptide or protein comprising at least one peptide or protein selected from the group consisting of Peptides/Proteins 1 to 106 of Table 1 in a second sample.

4. The method of claim 3 wherein said second sample contains a known amount and/or activity of said protease belonging to the POP-family.

5. The method of any one of claims 1 to 4 wherein the step of allowing a protease belonging to the POP-family present in the sample to contact a substrate for a protease belonging to the POP-family comprises the step of adding a substrate for a protease belonging to the POP-family to said sample.

6. The method of any one of claims 1 to 5 wherein the substrate is present in the sample in situ.

7. The method of any one of claims 1 to 6 wherein the sample is present in and/or derived from an organism.

8. The method of any one of claims 1 to 7 further comprising the step of allowing said protease belonging to the POP-family present in the sample to contact at least one compound and/or composition and determining whether said at least one compound and/or composition has an inhibitory, activating or no effect on said protease belonging to the POP-family.

9. A method of determining whether a compound or composition modifies the properties of a protease belonging to the POP-family comprising the steps of contacting a sample comprising said protease belonging to the POP-family with said compound or composition and a substrate for said protease belonging to the POP-family and determining the amount of at least one peptide or protein comprising at least one peptide or protein selected from the group consisting of Peptides/Protein 1 to 106 of Table 1.

10. A method of determining whether a compound or composition is effective against a disease or condition comprising the steps of contacting a sample comprising a protease belonging to the POP-family with said compound or composition and a substrate for a protease belonging to the POP-family and determining the amount of at least one peptide or protein comprising at least one peptide or protein selected from the group consisting of Peptides/Protein 1 to 106 of Table 1 wherein a compound or composition that has an inhibitory effect on the activity of said protease belonging to the POP-family indicates that the compound or composition is effective against said disease or condition.

11. The method of claim 10 further comprising the step of determining the inhibitory effect of at least one further compound or composition known to be effective against said disease or condition and comparing the inhibitory effects of said compound or composition the effectiveness of which against said disease or condition is to be determined with said at least one further compound or composition known to be effective against said disease or condition.

12. The method according to claim 10 or 11, wherein said disease or condition is selected from the group consisting of diabetes type 2, obesity, hyperlipidemia, osteoporosis, fertility disorders, cancer, angiogenesis, AIDS, inflammatory diseases, autoimmune diseases, and chronic rheumatoid arthritis.

13. The method of any one of claims 1 to 12 wherein the step of determining the amount of at least one peptide or protein comprises the step of determining the amount of at least one substrate for a protease belonging to the POP-family and/or at least one product formed by the activity of a protease belonging to the POP-family.

14. The method of any one of claims 1 to 13 wherein the substrate for said protease belonging to the POP-family comprises at least one peptide or protein selected from the group consisting of Peptides/Proteins 1 to 20 of Table 1.

15. The method of claim 13 wherein the product formed by the activity of a protease belonging to the POP-family comprises at least one peptide or protein selected from the group consisting of Peptides/Proteins 21 to 106 of Table 1.

16. The method of any one of claims 1 to 15 wherein the step of determining the amount of at least one peptide or protein comprising at least one peptide or protein selected from the group consisting of Peptides/Proteins 1 to 106 of Table 1 comprises determining the absolute or relative concentration of said at least one peptide or protein.

17. The method of any one of claims 1 to 16 wherein the step of determining the amount of at least one peptide or protein comprising at least one peptide or protein selected from the group consisting of Peptides/Proteins 1 to 106 of Table 1 is performed in a time dependent manner.

18. The method of claim 17 wherein the amount of said at least one peptide or protein is determined at a first time and at least a second time.

19. The method of any one of claims 1 to 18 wherein the step of determining the amount of at least one peptide or protein comprising at least one peptide or protein selected from the group consisting of Peptides/Proteins 1 to 106 of Table 1 comprises the step of determining a peptide or protein pattern resulting from allowing said protease belonging to the POP-family present in the sample to contact a substrate for said protease belonging to the POP-family or contacting said sample comprising a protease belonging to the POP-family with a substrate for said protease belonging to the POP-family.

20. The method of any one of claims 1 to 19 wherein said protease belonging to the POP-family is dipeptidyl peptidase 4 (DPP4) or dipeptidyl-protease 8 (DPP8).

21. A peptide or protein comprising at least one peptide or protein selected from the group consisting of Peptides/Proteins 1 to 106 of Table 1.

22. The peptide or protein according to claim 21 comprising a sequence selected from the group consisting of SEQ ID No: 1 to 9.

23. A peptide or protein generated by proteolysis of a peptide bond between Proline-Xaa in the consensus sequence N-terminus-Xaa-Proline-Xaa-C-terminus of a peptide or protein comprising at least one peptide or protein selected from the group consisting of Peptides/Proteins 1 to 106 of Table 1, wherein proline can be replaced by hydroxyproline, alanine, serine, glycine, valine, threonine or leucine and wherein Xaa represents any amino acid except proline, hydroxyproline or N-methyl glycine.

24. A nucleic acid which is selected from the group consisting of:
(a) a nucleic acid which comprises a nucleic acid sequence encoding a peptide or protein according to any one of claims 21 to 23 or derivative thereof,
(b) a nucleic acid which hybridizes with the nucleic acid of (a) under stringent conditions,
(c) a nucleic acid which is degenerate with respect to the nucleic acid of (a) or (b), and
(d) a nucleic acid which is complementary to the nucleic acid of (a), (b) or (c).
